# EUROPEAN PATENT APPLICATION

(11) **EP 4 219 675 A1**
(43) Date of publication of application: **02.08.2023**
(21) Application number: 21872530.7
(22) Date of filing: 24.09.2021
(51) Int. Cl.: C12M 1/02, C12M 1/34, C12M 3/02, C12N 5/071

(54) **CELL CULTURE APPARATUS AND METHOD FOR PRODUCING CELL GROUPS**

(30) Priority: 25.09.2020 JP 2020161197
(71) Applicant: Resonac Corporation, Tokyo 105-8518 (JP)
(72) Inventor: SUZUKI Yuma, Tokyo 100-6606 (JP); CHEN Shangwu, Tokyo 100-6606 (JP); OKANOJO Masahiro, Tokyo 100-6606 (JP); TAKAHASHI Ryosuke, Tokyo 100-6606 (JP)
(74) Representative: Winter, Brandl - Partnerschaft mbB
(86) International application number: PCT/JP2021/034944
(87) International publication number: WO 2022/065401

(57) **Abstract**

Provided are a cell culture apparatus capable of culturing cells with a high yield and time efficiency by efficiently advancing bead to bead transfer of the cells in a case where culture carriers are introduced into a suspension and cells are cultured on the culture carriers, and a method for manufacturing a cell group. A cell culture apparatus includes a culture container that accommodates a suspension containing at least cell complexes that are complexes of cells and culture carriers; a drive device that agitates the suspension; a measurement device that measures at least one of a cell distribution information value related to the cells and a carrier distribution information value related to the culture carriers in at least one partial region in the suspension; and a control device that controls driving of the drive device on the basis of at least one of the cell distribution information value and the carrier distribution information value measured by the measurement device.

## Description

### Technical Field

The present disclosure relates to a cell culture apparatus and a method for manufacturing a cell group.

### Background Art

In the field of biomedical research and clinical practice, a sample collected from a subject is cultured in vitro, and then a target sample (for example, cells, humoral factors, and the like) is sorted and recovered for use in subsequent research and treatment. For example, in the field of regenerative medicine, after cells collected from a subject proliferate in a culture medium, unintended cells or impurities are separated from the culture medium, and intended cells or humoral factors are recovered and used.

A cell culture process has involved a lot of manual operation. For example, in a case where culture carriers are introduced into a suspension and cells are cultured on the culture carriers, a technique in which new culture carriers are added into the suspension during a culture process, and the cells present on the originally introduced culture carriers are moved onto the new culture carriers to improve a yield of the cultured cells (in general, also referred to as "bead to bead transfer", or the like) is known. For example, Martial Hervy, et al., "Long Term Expansion of Bone Marrow-Derived hMSCs on Novel Synthetic Microcarriers in Xeno-Free, Defined Conditions" (https://journals.plos.org/plosone/article?id=10.1371/journal.pone.0092120). In this case, a confirmation operation such as whether the cells are moved smoothly onto the new culture carriers has been mainly performed manually.

In addition to the above, during the culture process, a suspension is collected at a predetermined time interval, and a concentration of cells contained in the suspension or the number of cells is observed, or measured, with a microscope or the like, such that a confirmation operation such as whether culture is performed smoothly or contamination of various bacteria or the like occurs is also mainly performed manually.

However, when a manual operation is frequently performed in the culture process, problems due to frequent collection of the suspension, such as a decrease in yield of the cultured cells and an increase in contamination risk of various bacteria occur. In recent years, various automation techniques have been proposed to reduce such problems.

For example, JP 2020-54234 A discloses a technique of acquiring statistical data of cells, or cell masses, contained in a suspension using an imaging unit.

JP 2010-99011 A and JP 2006-320226 A disclose a technique of measuring a distribution state, or dispersion state of cells in a culture container using an imaging apparatus and then controlling agitation of the culture container by a drive device according to the distribution state of the cells at the time of a cell seeding operation.

### Summary of Invention

### Technical Problem

However, in a case of suspension culture, or large scale culture, of cells, particularly, in a case of suspension culture in which cells are supported on culture carriers in a suspension, it is required to efficiently move the cells present on the culture carriers originally introduced into the suspension onto new culture carriers and further to reliably confirm the movement and to advance a culture process without a manual operation from the viewpoint of improving a yield of cultured cells, or a cell group, and time efficiency.

Therefore, according to various embodiments, there are provided a cell culture apparatus capable of culturing cells and a method for manufacturing a cell group with a high yield and time efficiency by efficiently advancing bead to bead transfer of the cells in a case where culture carriers are introduced into a suspension and the cells are cultured on the culture carriers .

### Solution to Problem

(1) A cell culture apparatus according to an aspect includes: a culture container that accommodates a suspension containing at least masses of cells and adherends to which the cells adhere; a drive device that agitates the suspension; a measurement device that measures a mass distribution information value related to the masses in at least one partial region in the suspension; and a control device that controls driving of the drive device on the basis of the mass distribution information value measured by the measurement device.
(2) The cell culture apparatus according to an aspect includes: a culture container that accommodates a suspension containing at least cell complexes that are complexes of cells and culture carriers; a drive device that agitates the suspension; a measurement device that measures at least one of a cell distribution information value related to the cells and a carrier distribution information value related to the culture carriers in the at least one partial region in the suspension; and a control device that controls driving of the drive device on the basis of at least one of the cell distribution information value and the carrier distribution information value measured by the measurement device.
(3) In the cell culture apparatus according to an aspect, in a case where additional culture carriers as new culture carriers are added into the culture container during a culture process of the cells, the control device controls driving of the drive device on the basis of at least one of a first change value calculated by comparing the cell distribution information value at a first time point after the addition of the additional culture carriers and the cell distribution information value at a second time point after the first time point, and a second change value calculated by comparing the carrier distribution information value at the first time point and the carrier distribution information value at the second time point.
(4) In the cell culture apparatus according to an aspect, in a case where additional culture carriers as new culture carriers are added into the culture container during a culture process of the cells, the control device controls driving of the drive device on the basis of a cell distribution information difference value calculated by a comparison operation between a first cell distribution information value in a first region in the at least one partial region after the addition of the additional culture carriers and a second cell distribution information value in a second region in the at least one partial region.
(5) In the cell culture apparatus according to an aspect, in a case where additional culture carriers as new culture carriers are added into the culture container during a culture process of the cells, the control device controls driving of the drive device on the basis of a carrier distribution information difference value calculated by a comparison operation between a first carrier distribution information value in a first region in the at least one partial region after the addition of the additional culture carriers and a second carrier distribution information value in a second region in the at least one partial region.
(6) In the cell culture apparatus according to an aspect, the measurement device includes a first measurement device including an electrode that forms an electric field in the at least one partial region and a capacitance measurement unit that measures a capacitance in the electric field, and the first measurement device measures the cell distribution information value in the at least one partial region on the basis of the capacitance.
(7) In the cell culture apparatus according to an aspect, the measurement device includes a second measurement device including an imaging unit that images the at least one partial region and acquires one or more images, and the second measurement device measures at least one of the cell distribution information value and the carrier distribution information value in the at least one partial region on the basis of the images.
(8) In the cell culture apparatus according to an aspect, the second measurement device measures the first cell distribution information value on the basis of the images in the first region, and measures the second cell distribution information value on the basis of the images in the second region.
(9) In the cell culture apparatus according to an aspect, the second measurement device measures the first carrier distribution information value on the basis of the images in the first region, and measures the second carrier distribution information value on the basis of the images in the second region.
(10) In the cell culture apparatus according to an aspect, the cell distribution information value is the number of living cells or a concentration of living cells among the cells in the suspension in the at least one partial region.
(11) In the cell culture apparatus according to an aspect, the carrier distribution information value is the number of culture carriers or a concentration of the culture carriers in the suspension in the at least one partial region.
(12) In the cell culture apparatus according to an aspect, the control device drives the drive device in a first mode until the first change value reaches a preset first predetermined value, and drives the drive device in a second mode different from the first mode after the first change value reaches the first predetermined value.
(13) In the cell culture apparatus according to an aspect, the control device drives the drive device in a first mode until the second change value reaches a preset second predetermined value, and drives the drive device in a second mode different from the first mode after the second change value reaches the second predetermined value.
(14) In the cell culture apparatus according to an aspect, the control device drives the drive device at an agitation speed in a first mode until the cell distribution information difference value reaches a preset predetermined value, and drives the drive device at an agitation speed in a second mode different from the first mode after the cell distribution information difference value reaches the predetermined value.
(15) In the cell culture apparatus according to an aspect, the control device drives the drive device at an agitation speed in a first mode until the carrier distribution information difference value reaches a preset predetermined value, and drives the drive device at an agitation speed in a second mode different from the first mode after the carrier distribution information difference value reaches the predetermined value.
(16) In the cell culture apparatus according to an aspect, the control device intermittently drives the drive device in the first mode.
(17) In the cell culture apparatus according to an aspect, the drive device is a stirrer or a shaker that stirs the suspension.
(18) In the cell culture apparatus according to an aspect, a volume of the culture container is 2.0 L or more.
(19) In the cell culture apparatus according to an aspect, the at least one partial region includes at least one of a central portion of the culture container in a depth direction in the suspension and a bottom surface portion of the culture container in the suspension.
(20) In the cell culture apparatus according to an aspect, the first region is a central portion of the culture container in a depth direction in the suspension, and the second region is a bottom surface portion of the culture container in the suspension.
(21) In the cell culture apparatus according to an aspect, the control device determines a time point at which the cell distribution information value reaches a predetermined set value after a start of culture of the cells as a time point at which additional culture carriers as new culture carriers are added into the culture container.
(22) A method for manufacturing a cell group according to an aspect is performed by a culture method, the culture method including: accommodating a suspension containing at least masses of cells and adherends to which the cells adhere in a culture container; agitating the suspension with a drive device; measuring a mass distribution information value related to the masses in at least one partial region in the suspension; determining an agitation speed of the drive device on the basis of the measured mass distribution information value; and outputting the determined agitation speed to the drive device.
(23) The method for manufacturing a cell group according to an aspect is performed by a culture method, the culture method including: accommodating a suspension containing at least cell complexes that are complexes of cells and culture carriers in a culture container; agitating the suspension with a drive device; measuring at least one of a cell distribution information value related to the cells and a carrier distribution information value related to the culture carriers in at least one partial region in the suspension; determining an agitation speed of the drive device on the basis of at least one of the measured cell distribution information value and carrier distribution information value; and outputting the determined agitation speed to the drive device.
(24) In the method for manufacturing a cell group according to an aspect, in a case where additional culture carriers as new culture carriers are added into the culture container during a culture process of the cells, the agitation speed is determined on the basis of at least one of a first change value calculated by comparing the cell distribution information value at a first time point after the addition of the additional culture carriers and the cell distribution information value at a second time point after the first time point, and a second change value calculated by comparing the carrier distribution information value at the first time point and the carrier distribution information value at the second time point.
(25) In the method for manufacturing a cell group according to an aspect, in a case where additional culture carriers as new culture carriers are added into the culture container during a culture process of the cells, the agitation speed is determined on the basis of a cell distribution information difference value calculated by a comparison operation between a first cell distribution information value in a first region among a plurality of regions after the addition of the additional culture carriers and a second cell distribution information value in a second region among the plurality of regions.
(26) In the method for manufacturing a cell group according to an aspect, in a case where additional culture carriers as new culture carriers are added into the culture container during a culture process of the cells, the agitation speed is determined on the basis of a carrier distribution information difference value calculated by a comparison operation between a first carrier distribution information value in a first region among a plurality of regions after the addition of the additional culture carriers and a second carrier distribution information value in a second region among the plurality of regions.

### Advantageous Effects of Invention

According to various embodiments, it is possible to provide a cell culture apparatus capable of culturing cells and a method for manufacturing a cell group with a high yield and time efficiency by efficiently advancing bead to bead transfer of the cells in a case where culture carriers are introduced into a suspension and cells are cultured on the culture carriers.

### Brief Description of Drawings

Fig. 1 is a schematic view schematically illustrating a configuration of a cell culture apparatus according to an embodiment.
Fig. 2 is a schematic view schematically illustrating a configuration in a case where a first measurement device is used as a measurement device in the cell culture apparatus according to an embodiment.
Fig. 3 is an enlarged view of a region surrounded by a dotted line in Fig. 2, and is a schematic view schematically illustrating an enlarged part of the first measurement device.
Fig. 4 is a schematic view schematically illustrating an example of an aspect of another drive device different from the drive device illustrated in Figs. 1 and 2 in the cell culture apparatus according to an embodiment.
Fig. 5 is a block diagram schematically illustrating an example of a function of a control device according to an embodiment.
Fig. 6 is a view schematically illustrating an example of a relationship between a determination result by a determination unit of the control device and an agitation speed of a drive device 20 that is determined by an output unit of the control device according to an embodiment.
Fig. 7 is a view schematically illustrating an example of a relationship between a determination result by the determination unit of the control device and an agitation speed of the drive device 20 that is determined by the output unit of the control device according to an embodiment.
Fig. 8 is a flowchart showing an example of a part of an operation performed in the cell culture apparatus according to an embodiment.
Fig. 9 is a view illustrating changes in capacitance and number of living cells with a lapse of time in relation to a proliferation status of cells by the cell culture apparatus according to an embodiment.
Fig. 10 is a view illustrating changes in capacitance and number of living cells with a lapse of time in relation to a proliferation status of cells by the cell culture apparatus according to an embodiment.
Fig. 11 is a schematic view schematically illustrating a configuration in a case where a second measurement device is used as a measurement device in a cell culture apparatus according to Modification 1.
Fig. 12 is a view illustrating an example of an image captured by an imaging unit in a cell culture apparatus according to Modification 2.
Fig. 13 is a schematic view schematically illustrating a configuration of a cell culture apparatus according to Modification 3.
Fig. 14 is a flowchart showing an example of a series of almost all operations performed in the cell culture apparatus.
Fig. 15 is a flowchart showing an operation related to a bead to bead transfer mode of the cell culture apparatus according to a first variation.
Fig. 16 is a flowchart showing an operation related to a bead to bead transfer mode of the cell culture apparatus according to a second variation.
Fig. 17 is a flowchart showing an operation related to a bead to bead transfer mode of the cell culture apparatus according to a third variation.
Fig. 18 is a flowchart showing an operation related to a bead to bead transfer mode of the cell culture apparatus according to a fourth variation.

### Description of Embodiments

Hereinafter, various embodiments will be described with reference to the accompanying drawings. Constituent elements common in the drawings are denoted by the same reference numerals. It should be noted that the components illustrated in a certain drawing may be omitted in another drawing for convenience of description. It should be noted that the accompanying drawings are not necessarily to scale.

### 1. Configuration of cell culture apparatus

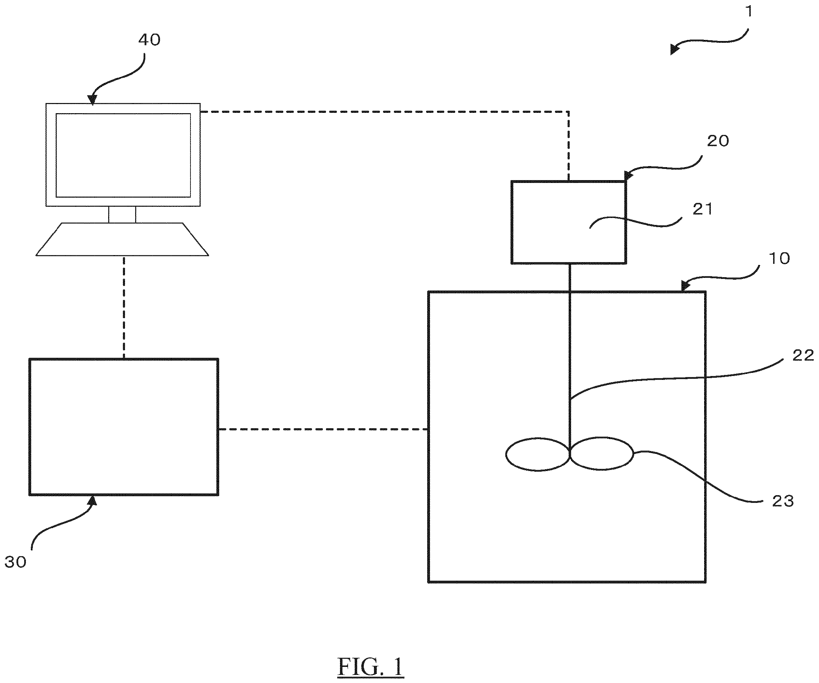

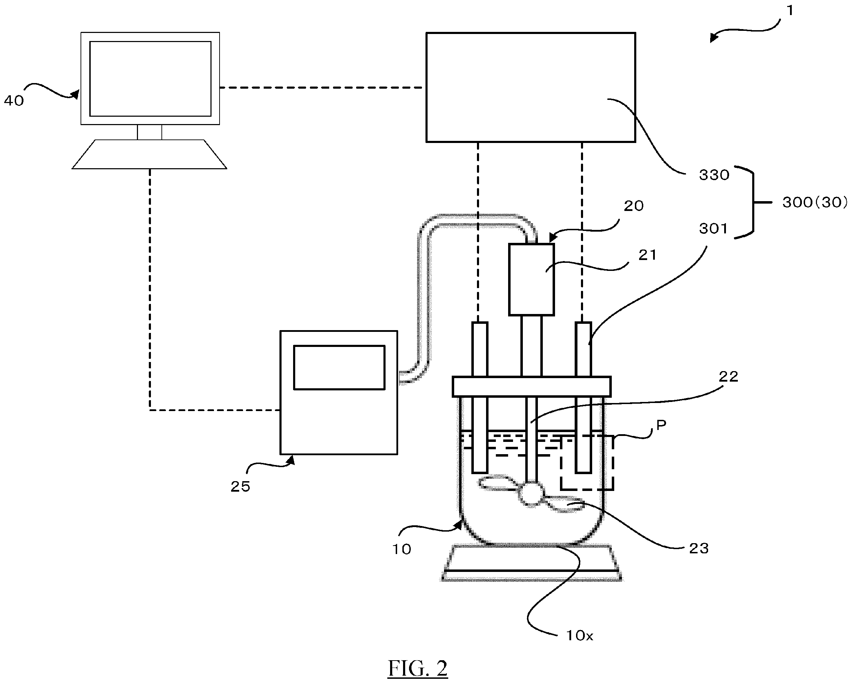

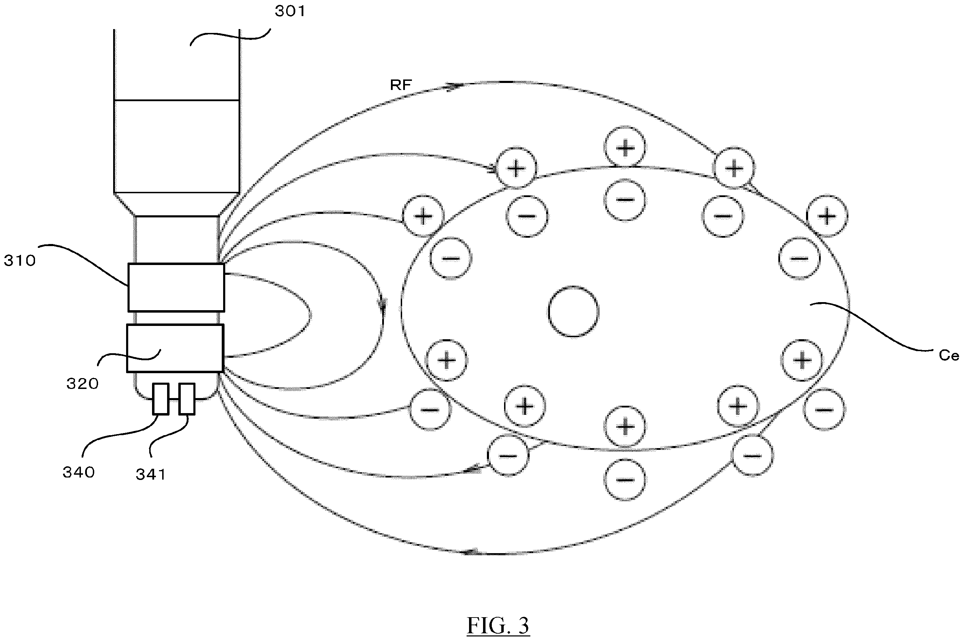

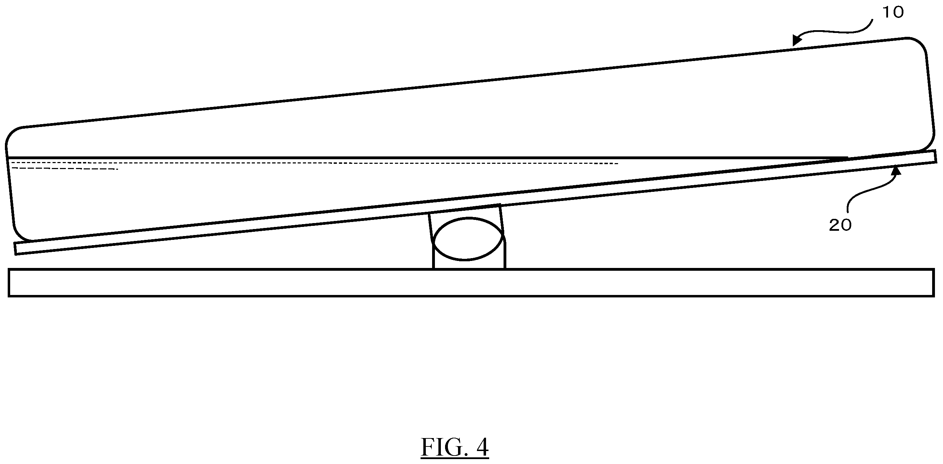

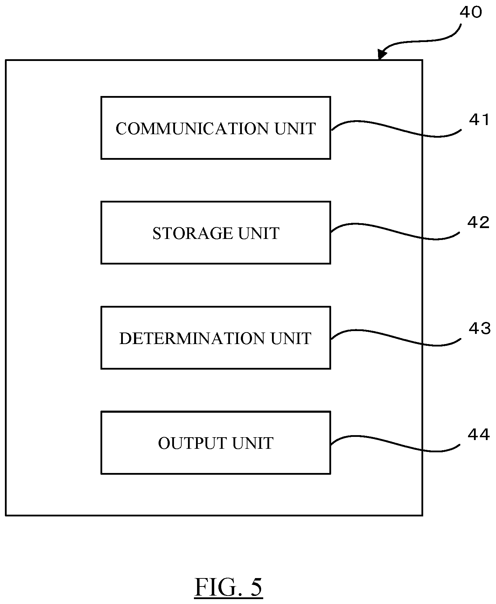

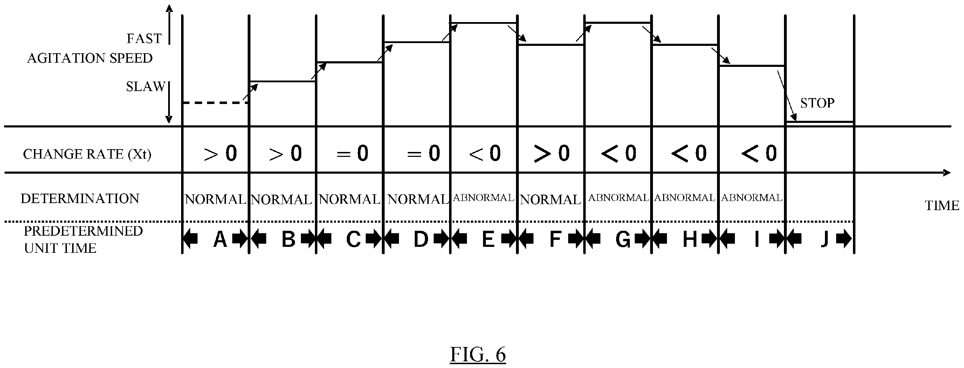

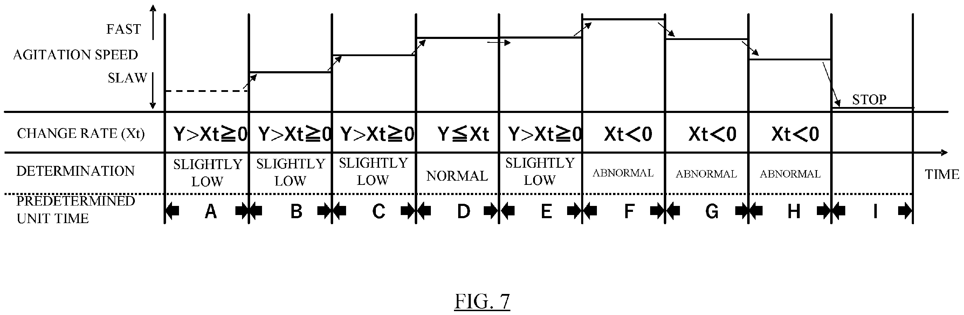

An overview of an overall configuration of a cell culture apparatus according to an embodiment will be described with reference to Figs. 1 to 7. Fig. 1 is a schematic view schematically illustrating a configuration of a cell culture apparatus 1 according to an embodiment. Fig. 2 is a schematic view schematically illustrating a configuration in a case where a first measurement device 300 is used as a measurement device 30 in the cell culture apparatus 1 according to an embodiment. Fig. 3 is an enlarged view of a region P surrounded by a dotted line in Fig. 2, and is a schematic view schematically illustrating an enlarged part of the first measurement device 300. Fig. 4 is a schematic view schematically illustrating an example of an aspect of another drive device 20 different from a drive device 20 illustrated in Figs. 1 and 2 in the cell culture apparatus 1 according to an embodiment. Fig. 5 is a block diagram schematically illustrating an example of a function of a control device 40 according to an embodiment. Figs. 6 and 7 are views schematically illustrating an example of a relationship between a determination result by a determination unit 43 of the control device 40 and an agitation speed of the drive device 20 that is determined by an output unit 44 of the control device 40 according to an embodiment.

Cells to be cultured in the cell culture apparatus 1 according to an embodiment are not particularly limited as long as they are cells that can be subjected to suspension culture, and are preferably adherent cells that can be cultured by adhering to culture carriers. As long as the cells can adhere to the culture carriers to form complexes of one or a plurality of cells and culture carriers, the plurality of cells may come into contact with each other during suspension culture to form aggregates, and may be suspended in the suspension as a single cell during culture. In the present disclosure, the "single cell" means one independent cell. In the present disclosure, the "cell masses that are aggregates of a plurality of cells" mean masses formed by cells coming into contact with each other, and include, but are not limited to, spheroids that are stereoscopically formed in a cell proliferation phase, masses formed by two or more cells collected by other factors, and the like.

The cells are preferably cells derived from an animal, and more preferably cells derived from a mammal. Examples of the mammal include a human, a monkey, a chimpanzee, a cow, a pig, a horse, a sheep, a goat, a rabbit, a rat, a mouse, a guinea pig, a dog, and a cat. The cells may be, for example, cells derived from tissues such as skin, liver, kidney, muscle, bone, blood, or nervous tissues. Usually, one kind of cells may be subjected to culture alone, or two or more kinds of cells may be subjected to culture in combination. The cells may be primary cells from tissues or cell lines established by immortalization.

Examples of the adherent cells include somatic cells and stem cells. Examples of the somatic cells include endothelial cells, epidermal cells, epithelial cells, cardiomyocytes, myoblasts, neuronal cells, bone cells, osteoblasts, fibroblasts, adipocytes, hepatocytes, renal cells, pancreatic cells, adrenal cells, periodontal ligament cells, gingival cells, periosteal cells, skin cells, dendritic cells, and macrophages.

In an embodiment, the adherent cells may be stem cells. Examples of the stem cells include somatic stem cells such as mesenchymal stem cells, hematopoietic stem cells, neural stem cells, bone marrow stem cells, and reproductive stem cells, and the stem cells can be mesenchymal stem cells or bone marrow stem cells. The mesenchymal stem cells mean somatic stem cells that are present in various tissues of a human body and can be differentiated into all or some of mesenchymal cells such as osteoblasts, chondrocytes, and adipocytes in a broad sense. The stem cells may further include induced pluripotent stem cells, or iPS cells and embryonic stem cells, or ES cells.

The suspension can be obtained by suspending these cells in a culture medium corresponding to the type of cells. A concentration of the cells in the suspension at a start of culture can be, for example, 1 × 10³ to 2 × 10⁵ cells/mL, 5 × 10³ to 1 × 10⁵ cells/mL, or 1 × 10⁴ to 5 × 10⁴ cells/mL.

The cell culture apparatus 1 according to an embodiment mainly includes a culture container 10, a drive device 20, a measurement device 30, and a control device 40, and may include other devices. For example, a heater or the like for heating the suspension accommodated in the culture container to a predetermined temperature may be separately provided. Although the measurement device 30 and the control device 40 are separately provided in Fig. 1, the present disclosure is not limited thereto, and at least a part of the measurement device 30 and the control device 40 may be integrally formed. Hereinafter, details of main components of the cell culture apparatus 1 will be described.

### 1-1. Culture container 10 and suspension in culture container 10

As the culture container 10 according to an embodiment, a generally known bioreactor can be used. A volume of the culture container 10 can be, for example, 0.3 L or more, 0.5 L or more, 1 L or more, 2 L or more, 5 L or more, 8 L or more, or 10 L or more, in order to enable suspension culture and mass culture. An upper limit value of the volume of the culture container 10 is not particularly limited, and can be, for example, 50 L or less, 40 L or less, or 30 L or less, from the viewpoint of efficiency and economic feasibility, but may be 80 L or more, 90 L or more, 100 L or more, or more than this.

A shape of the culture container 10 is not particularly limited, and for example, a cylindrical, prismatic, or bag-shaped culture container can be used. It is preferable that a side surface and a bottom surface of the culture container 10 have a material, a color, and a shape through which irradiation light emitted from an imaging unit 360 described below passes in order to image the suspension by the imaging unit 360. For the same reason, other components, for example, the heater described above and the like, disposed around the culture container 10 are preferably arranged on at least one of the side surface and the bottom surface of the culture container 10 at a position different from a route of the irradiation light emitted from the imaging unit 360, or a position deviated from the route.

A suspension containing cells is accommodated in the culture container 10 and subjected to suspension culture. Various culture carriers corresponding to the type of cells to be cultured are also introduced into the suspension. As the culture carriers, for example, microbeads known as microcarriers used for suspension culture of cells and having a diameter of several hundred µm can be used. Therefore, when target cells are cultured, the cells are supported on or brought into contact with microbeads suspending in a culture medium to proliferate, such that the cells can be obtained with a large culture area and a high density.

In a case of culture using culture carriers, driving of the drive device 20 may be stopped during a certain period of time, may be intermittently driven, or may be continuously driven according to the type, state, or the like of the cells at the time of initial addition of the culture carriers into the culture container 10 or immediately after the addition of the culture carriers. Details of the intermittent driving and the continuous driving will be described below.

A material of the culture carrier may be, for example, an organic substance, an inorganic substance, or a composite material thereof, and may be either dissolvable or insoluble. Examples of the organic substance include, but are not limited to, synthetic polymers such as polystyrene, polyester, polyurethane, polyethylene, polypropylene, polyvinyl alcohol, a (meth)acrylic polymer, a (meth)acrylamide polymer, a silicone polymer, an epoxy resin, and a urethane resin, and natural polymers such as cellulose, dextran, collagen, polygalacturonic acid, polyalginic acid, gelatin, and protein. Examples of the inorganic substance include, but are not limited to, glass, ceramic, a metal, an alloy, and a metal oxide. As the culture carriers of an aspect, culture carriers containing polystyrene may be used.

In the culture carriers, cationic functional groups may be introduced into a surface of the culture carrier in order to improve a carrying property or adhesiveness with cells. Examples of the cationic functional group include groups having a substituted or unsubstituted amino group such as a dimethylamino group, a diethylamino group, and an amino group. A cell adhesive polymer may be disposed on the surface of the culture carrier from the viewpoint of promoting adhesion of the cells. As the cell adhesive polymer, collagen, gelatin, alginic acid, Matrigel (trademark) (BD Biosciences), hyaluronic acid, laminin, fibronectin, vitronectin, elastin, heparan sulfate, dextran, dextran sulfate, chondroitin sulfate, or the like may be disposed.

Examples of a shape of the culture carrier include a spherical shape, a flat shape, a cylindrical shape, a plate shape, and a prismatic shape. It is preferable that the culture carriers include spherical culture carriers. The culture carriers may be porous culture carriers having pores therein, or may be culture carriers having no cells therein.

An average particle size (D50) of the culture carriers is, for example, 50 to 1,000 µm, preferably 100 to 500 µm, and more preferably 150 to 250 µm, from the viewpoint of promoting cell proliferation. The average particle size of the culture carriers is a value measured as a median diameter (D50) in physiological saline or a culture medium. The average particle size of the culture carriers can be measured by a laser diffraction scattering type particle size distribution measurement device.

A concentration of the culture carriers in the suspension can be appropriately adjusted on the basis of the shape, size, surface area, and the like of the culture carrier, and can be, for example, 0.01 to 100 g/L, 0.5 to 50 g/L, or 1 to 20 g/L.

When proliferation or suspension culture of the cells proceeds with a lapse of time in the culture container 10, cell complexes that are complexes of cells and culture carriers are formed, and the cells proliferate on the cell complexes or on the culture carriers. The suspension may contain not only cell complexes, cell masses or aggregates formed by aggregation of a plurality of cells, culture carriers (including additional culture carriers to be described below) to which cells are not adhered, and the like.

In the present disclosure, the mass is formed of cells and adherends to which the cells adhere. The adherends may include, for example, cells, culture carriers, and the like, and the masses may include, for example, the cell masses and cell complexes described above, and the like. The mass may be a suspending single cell. In this regard, in the present disclosure, the terms of a cell distribution information value and a carrier distribution information value to be described below can be read as a mass distribution information value, and the terms of a cell distribution information difference value and a carrier distribution information difference value to be described below can be read as a mass distribution information difference value.

### 1-2. Drive device 20

The drive device 20 according to an embodiment can be used without particular limitation as long as it agitates the suspension accommodated in the culture container 10. As the drive device 20, for example, as illustrated in Figs. 1 and 2, a stirrer including a motor 21, a shaft 22 connected to a rotating shaft (not illustrated) of the motor 21, and stirring blades 23 connected to a distal end of the shaft 22 can be used.

As the drive device 20, for example, a generally known shaker as illustrated in Fig. 4 may be used to shake the culture container 10 from the outside by the shaker so as to agitate the suspension. Therefore, in the present disclosure, the "agitation" can include all modes of motion for agitating the suspension, such as stirring and shaking. Similarly, in the present disclosure, the "agitation speed" can include speeds related to all modes of motion for agitating the suspension, such as a stirring speed and a shaking speed.

The drive device 20 is separately provided with a receiving unit (not illustrated) that receives a command from a control device 40 to be described below that controls an agitation speed or stirring speed of a drive source, or the motor 21 illustrated in Fig. 1 in a case where a stirrer is used as the drive device 20. Therefore, the drive device 20 can transmit the agitation speed corresponding to the command from the control device 40 to the drive source.

### 1-3. Measurement device 30

The measurement device 30 according to an embodiment measures or calculates at least one of a cell distribution information value related to the cells and a carrier distribution information value related to the culture carriers in at least a partial region in the suspension in the culture container 10 while the culture is in progress.

The cell distribution information value can also be referred to as the number of living cells or a concentration of living cells in the suspension in at least a partial region, but is not limited thereto. That is, the cell distribution information value may be a value indicating the number of living cells distributed in the suspension in at least a partial region, and in some cases, may be the number of cell masses formed by aggregation of a plurality of cells.

On the other hand, the carrier distribution information value can also be referred to as the number of culture carriers or a concentration of the culture carriers in the suspension in at least a partial region, but is not limited thereto. That is, the carrier distribution information value may be a value indicating the number of culture carriers distributed in the suspension in at least a partial region.

As the measurement device 30 of the cell culture apparatus 1 according to an embodiment, for example, the first measurement device 300 as illustrated in Fig. 2 and the like can be used. A first measurement device 300 to be described in detail below can measure only the cell distribution information value, and a second measurement device 350 according to Modification 1 to be described below can measure both the cell distribution information value and the carrier distribution information value. Which of the first measurement device 300 and a second measurement device 350 to be described below is used as the measurement device 30 may be appropriately determined on the basis of the type of cells to be cultured, the size of the culture container 10, and the like, but the first measurement device 300 is preferable in a case where the number of living cells or a concentration of living cells is directly observed or managed, and the second measurement device 350 is preferable in a case where cell masses or cell complexes are directly observed or managed.

As illustrated in Figs. 2 and 3, the first measurement device 300 can include a substantially rod-shaped main part 301 that is inserted into the suspension in the culture container 10 in a region P surrounded by the dotted line in Fig. 2, an electrode 310 that is provided on the main part 301 and oscillates an electric signal RF of a predetermined frequency to form an electric field in the suspension in the region P, a capacitance measurement unit 320 that measures a capacitance or electrostatic capacitance in the electric field on the basis of the amount of living cells Ce polarized in the formed electric field, and a main unit 330 that calculates a cell distribution information value in the region P, or specifically, the number of living cells or a concentration of living cells in the region P on the basis of the capacitance measured by the capacitance measurement unit 320. Furthermore, at least one or both of a conductivity measurement unit 340 that measures a conductivity of the suspension in the region P and a pH measurement unit 341 that measures a pH of the suspension in the region P may be disposed in the main part 301. As the conductivity measurement unit 340 and the pH measurement unit 341, generally known measurement units can be used. As illustrated in Fig. 2, in the first measurement device 300, a plurality of main parts 301 (two in Fig. 2) may be provided, and accordingly, a plurality of electrodes 310, capacitance measurement units 320, conductivity measurement units 340, pH measurement units 341, and the like may also be provided.

The frequency of the electric signal RF oscillated by the electrode 310 is not particularly limited, and for example, a frequency of 50 kHz to 20 MHz, preferably 100 kHz to 1 MHz, and more preferably 400 kHz to 600 kHz can be used. It is preferable that the electric signals of a plurality of types of frequencies are oscillated by the electrode 310. Various types of frequencies of the electric signal RF are oscillated by the electrode 310, such that the number of living cells or the concentration of living cells for each living cell with various particle sizes can be measured. Therefore, the number of living cells or the concentration of living cells contained in the suspension in the region P can be exactly measured.

A location of the region P in the suspension where an electric field is formed by the electrode 310 and a capacitance is measured by the capacitance measurement unit 320 may be any position in a depth direction inside the culture container 10 as long as it is below a liquid surface of the suspension when the suspension is accommodated in the culture container 10. For example, as illustrated in Figs. 2 and 3, the location of the region P may be a central portion in the depth direction in the culture container 10, or may be a bottom surface portion 10x in the culture container 10. The number of regions P in the suspension may be provided at only one position or a plurality of positions in the culture container 10. When a plurality of regions P are provided in the culture container 10, the regions P may be disposed at different positions or at the same position in the depth direction in the culture container 10. From the viewpoint of accuracy of determination by a control device 40 to be described below, a portion where a capacitance is measured (by the capacitance measurement unit 320) is preferably measured at a plurality of locations in the culture container 10. In a case where a capacitance is measured at a plurality of locations, or a plurality of regions, for example, regions Q1 to Q4 to be described below in the suspension in the culture container 10, the first measurement device 300 may be designed to have a plurality of electrodes 310 and a plurality of capacitance measurement units 320 (and the main parts 301 corresponding thereto) corresponding to the plurality of locations.

It is known that dead cells, or cells whose cell membranes are ruptured are not polarized in an electric field. Therefore, the first measurement device 300 can accurately calculate the number of living cells or the concentration of living cells in the suspension. Since a device itself that measures the number of living cells or the concentration of living cells using a capacitance (there is a proportional relationship between the capacitance and the number of living cells) is already known (for example, see Harriet E. Cole et al., "The Application of Dielectric Spectroscopy and Biocalorimetry for the Monitoring of Biomass in Immobilized Mammalian Cell Cultures", Processes 2015, 3, 384-405, and the like), a further detailed description of the first measurement device 300 will be omitted.

The cell distribution information value, or the number of living cells or the concentration of living cells calculated by the main unit 330 of the first measurement device 300 is transmitted to a control device 40 to be described below at a predetermined time interval, for example, an interval of a predetermined unit time.

### 1-4. Control device 40

The control device 40 according to an embodiment controls driving of the drive device 20 on the basis of at least one of the cell distribution information value and the carrier distribution information value measured by the measurement device 30. As described below, the control device 40 can also be regarded as a monitoring device since it also has a function of monitoring the progress of the bead to bead transfer of the cells on the cell complexes onto the additional culture carriers. The control device 40 according to an embodiment can use, for example, general hardware in which a central processing unit or CPU, a main storage device, an input/output interface, an input device, an output device, and the like are connected by a data bus or the like. As illustrated in Fig. 1, the control device 40 may be provided separately from the measurement device 30 as a terminal device, or at least a part of the measurement device 30 (for example, the main unit 330 in the first measurement device 300) and the control device 40 may be integrally configured.

As illustrated in Fig. 5, functions of the control device 40 according to an embodiment are mainly composed of a communication unit 41, a storage unit 42, a determination unit 43, and an output unit 44.

The communication unit 41 receives the cell distribution information value from the measurement device 30, or the first measurement device 300 in an embodiment and transmits the received cell distribution information value to the storage unit 42. As described below, the communication unit 41 can transmit the agitation speed of the drive device 20 determined or calculated by the output unit 44 on the basis of a determination result of the determination unit 43 to the drive device 20 as a control command to the drive device 20 (the drive device 20 has a function of receiving a control command from the communication unit 41).

The storage unit 42 can store the cell distribution information value received from the communication unit 41, the control command to the drive device 20, a threshold to be described below, and the like.

In a case of a mode for performing normal cell culture (in the present disclosure, also referred to as a "culture mode" or a "second mode"), the determination unit 43 monitors a change rate in the cell distribution information value per predetermined unit time (hereinafter, also simply referred to as a "change rate") on the basis of the cell distribution information value received by the communication unit 41 (stored by the storage unit 42) from the measurement device 30. Furthermore, the determination unit 43 compares the change rate with a preset threshold for the change rate, and determines a relationship between the change rate and the threshold every predetermined unit time. For example, the determination unit 43 determines whether the change rate is equal to or greater than the threshold or less than the threshold. In calculating the change rate, for example, when the predetermined unit time is set to 1 hour or 60 minutes, the change rate in the cell distribution information value for 1 hour can be calculated by a comparison operation between a cell distribution information value at the start and a cell distribution information value at the end for 1 hour (that is, after 60 minutes from the start time). For example, when the predetermined unit time is set to 1 hour or 60 minutes, a cell distribution information value may be acquired a total of seven times every 10 minutes from the start for 1 hour (for example, at the start, after 10 minutes, after 20 minutes, after 30 minutes, after 40 minutes, after 50 minutes, and at the end), a change rate in the cell distribution information value from the start to 10 minutes, a change rate in the cell distribution information value from 10 minutes to 20 minutes, a change rate in the cell distribution information value from 20 minutes to 30 minutes, a change rate in the cell distribution information value from 30 minutes to 40 minutes, a change rate in the cell distribution information value from 40 minutes to 50 minutes, and a change rate in the cell distribution information value from 50 minutes to the end may be calculated, and an average value of the seven times in total may be used as a change rate in the cell distribution information value per predetermined unit time. As described above, since there is a proportional relationship between the capacitance and the number of living cells, a change rate in the capacitance per predetermined unit time may be used instead of the change rate in the cell distribution information value per predetermined unit time. In this case, the measurement device 30 may transmit information regarding the measured capacitance or capacitance value to a control device 40 to be described below at a predetermined time interval (for example, an interval of a predetermined unit time) together with the cell distribution information value calculated by the main unit 330 or instead of the cell distribution information value calculated by the main unit 330 (therefore, in the present disclosure, the "change rate in the cell distribution information value" also includes the "change rate in the capacitance").

The predetermined unit time related to the change rate is not particularly limited, and may be appropriately set, for example, between 10 minutes and 24 hours. However, the predetermined unit time is preferably, for example, 30 minutes to 1 hour, from the viewpoint of finely controlling the agitation speed of the drive device 20 to maximize the yield and the efficiency of the cells to be cultured (the aggregate of cells to be cultured is also referred to as a "cell group" in the present disclosure).

The threshold described above is set to, for example, "0%" with respect to the change rate described above (the threshold in this case is also referred to as a "first threshold"). The change rate in 0% means that the cell distribution information value to be measured is constant in a predetermined unit time (at the start and the end of the predetermined unit time). A case where the change rate is more than 0% means that the cell distribution information value is increased, and a case where the change rate is less than 0% means that the cell distribution information value is decreased. For example, the determination unit 43 can determine that proliferation of cells is "normal" when the change rate is 0% or more (the first threshold or more), and can determine that proliferation of cells is "abnormal" when the change rate is less than 0% (less than the first threshold). The specific determination contents of "normal" and "abnormal" may be appropriately changed.

As the threshold, in addition to the first threshold, another threshold (in this case, another threshold is also referred to as a "second threshold") may be further set to a value greater than 0%. For example, the second threshold can be set to a value in which the change rate is in a range of 10% to 90%. As described above, the second threshold is set in addition to the first threshold, such that it is possible to increase variations in determination by the determination unit 43 and to finely control the agitation speed of the drive device 20. That is, for example, the determination unit 43 can determine that proliferation of cells is "normal" when the change rate is equal to or greater than the second threshold, can determine that proliferation of cells is "slightly low" when the change rate is equal to or greater than the first threshold and less than the second threshold, and can determine that proliferation of cells is "abnormal" when the change rate is less than the first threshold. In the case, the specific determination contents of "normal", "slightly low", and "abnormal" may be appropriately changed.

The output unit 44 determines a value of the agitation speed of the drive device 20 (in a case where a stirrer is used as the drive device 20, a stirring speed) on the basis of the determination result of the determination unit 43 described above. A relationship between the determination result of the determination unit 43 and the agitation speed of the drive device 20 determined by the output unit 44 will be described with reference to Figs. 6 and 7. In Figs. 6 and 7, it should be understood that all of lengths of the predetermined unit times (a predetermined unit time A to a predetermined unit time J in Fig. 6, and a predetermined unit time A to a predetermined unit time I in Fig. 7) are constant (for example, 1 hour as described above), and the end time and the start time of adjacent predetermined unit times are substantially the same (that is, the end time of the predetermined unit time A and the start time of the predetermined unit time B are substantially the same).

First, a case where only the first threshold is set as a threshold for the change rate (Xt) will be described with reference to Fig. 6. In this case, when the determination unit 43 determines that it is "normal" (the change rate is equal to or greater than the first threshold "0") as described above at the end of a certain predetermined unit time (for example, the predetermined unit time A in Fig. 6), the output unit 44 outputs a new agitation speed (note that a time point at which a new agitation speed is output is the start time of the next predetermined unit time B) so as to increase the agitation speed of the drive device 20 at the time of the determination (that is, at the time of the predetermined unit time A).

On the other hand, when the determination unit 43 determines that it is "abnormal" (the change rate is less than the first threshold "0") as described above at the end of a certain predetermined unit time (for example, the predetermined unit time E in Fig. 6), the output unit 44 outputs a new agitation speed (note that a time point at which a new agitation speed is output is the start time of the next predetermined unit time F) so as to decrease the agitation speed of the drive device 20 at the time of the determination (that is, at the time of the predetermined unit time E). As a result of decreasing the agitation speed, when the change rate becomes equal to or greater than the first threshold (the change rate is the first threshold "0") again, the determination unit 43 determines that it is "normal", and thus, similarly to the above case, a new agitation speed for increasing the agitation speed of the drive device 20 is output (see a predetermined unit time F and a predetermined unit time G in Fig. 6).

In a case where the determination unit 43 determines that it is "abnormal" a plurality of times in a row every predetermined unit time (that is, the change rate is less than the first threshold), the output unit 44 outputs an agitation speed 0 to stop driving of the drive device 20 (see a predetermined unit time G to a predetermined unit time J in Fig. 6). In a case where the determination of the predetermined unit time is determined to be "abnormal" at a plurality of times in a row, it is considered that a problem due to occurrence of contamination such as various bacteria in the culture container 10, damage to living cells caused by an excessively fast agitation speed of the drive device 20, or the like may occur, and thus it is required to immediately stop culture of cells. The "plurality of times in a row" described above may mean that the determination every predetermined unit time is continuous two times, continuous three times, or continuous four times, and may be appropriately set according to the type of cells or culture conditions. In an embodiment, the problem as described above may be grasped on the basis of the measured value of at least one of the conductivity in the suspension measured by the conductivity measurement unit 340 and the pH of the suspension measured by the pH measurement unit 341.

Next, a case where a second threshold Y is set as a threshold for the change rate (Xt) in addition the first threshold will be described with reference to Fig. 7. In this case, when the determination unit 43 determines that it is "slightly low" (the change rate Xt is "Y > Xt ≥ 0") as described above at the end of a certain predetermined unit time (for example, the predetermined unit time A in Fig. 7), the output unit 44 outputs a new agitation speed (note that a time point at which a new agitation speed is output is the start time of the next predetermined unit time B) so as to increase the agitation speed of the drive device 20 at the time of the determination (at the time of the predetermined unit time A).

Next, when the determination unit 43 determines that it is "normal" (the change rate Xt is "Y ≤ Xt") at the end of a certain predetermined unit time (for example, the predetermined unit time D in Fig. 7), the output unit 44 maintains the agitation speed at the time of determination (at the time of the predetermined unit time D).

Furthermore, when the determination unit 43 determines that it is "abnormal" (the change rate Xt is "Xt < 0") as described above at the end of a certain predetermined unit time (for example, the predetermined unit time F in Fig. 7), the output unit 44 outputs a new agitation speed (note that a time point at which a new agitation speed is output is the start time of the next predetermined unit time G) so as to decrease the agitation speed at the time of the determination (at the time of the predetermined unit time F). Also in the case illustrated in Fig. 7, as in the case of Fig. 6, in a case where it is continuously determined as "abnormal" a plurality of times during a predetermined period, the output unit 44 outputs the agitation speed 0 to stop the drive device 20.

In the case illustrated in Figs. 6 and 7, a specific agitation speed and a degree of increase or decrease in agitation speed of the drive device 20 may be appropriately set according to the type of cells, the volume of the culture container 10, and the like. However, when the agitation speed of the drive device 20 is too fast, there is a risk of damaging cells, and thus a specific agitation speed can be set within a range of 10 mm/s to 100 mm/s, preferably 20 mm/s to 80 mm/s, and more preferably 30 mm/s to 60 mm/s. The agitation speed at this time means a rotation speed of the stirring blade 23 in a case where a stirrer is used as the drive device 20, and the agitation speed may be converted into a rotation speed as a movement distance of an outer peripheral end of the stirring blade 23.

Hereinabove, the details of the communication unit 41, the storage unit 42, the determination unit 43, and the output unit 44 of the control device 40 have been described focusing on the culture mode (second mode).

In a case other than the culture mode, that is, a description focusing on details of the communication unit 41, the storage unit 42, the determination unit 43, and the output unit 44 of the control device 40 related to the bead to bead transfer mode (first mode) will be described below.

### 2. Operations of cell culture apparatus 1 in culture mode

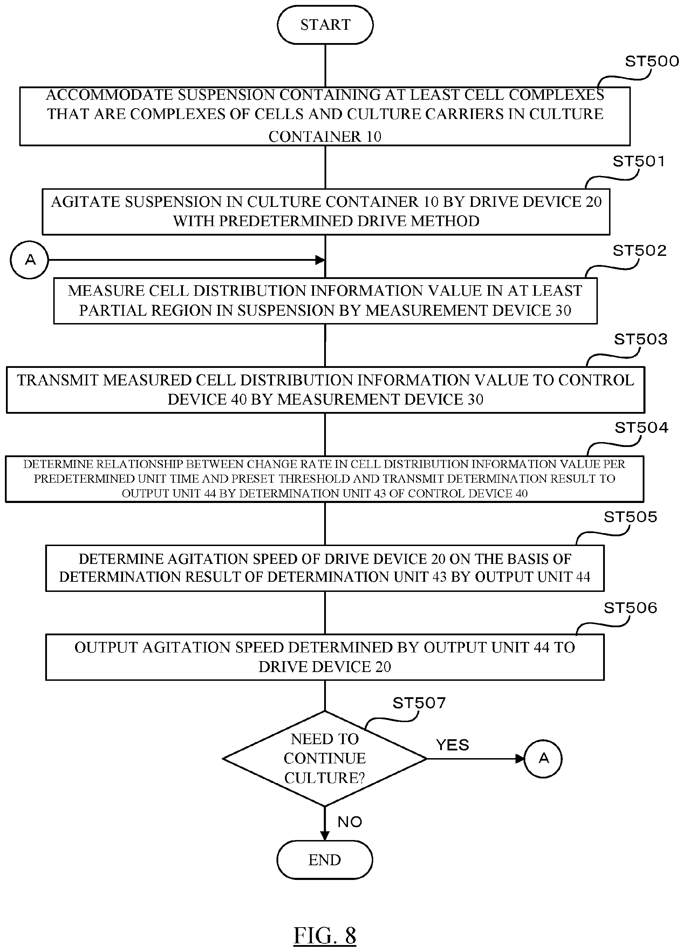

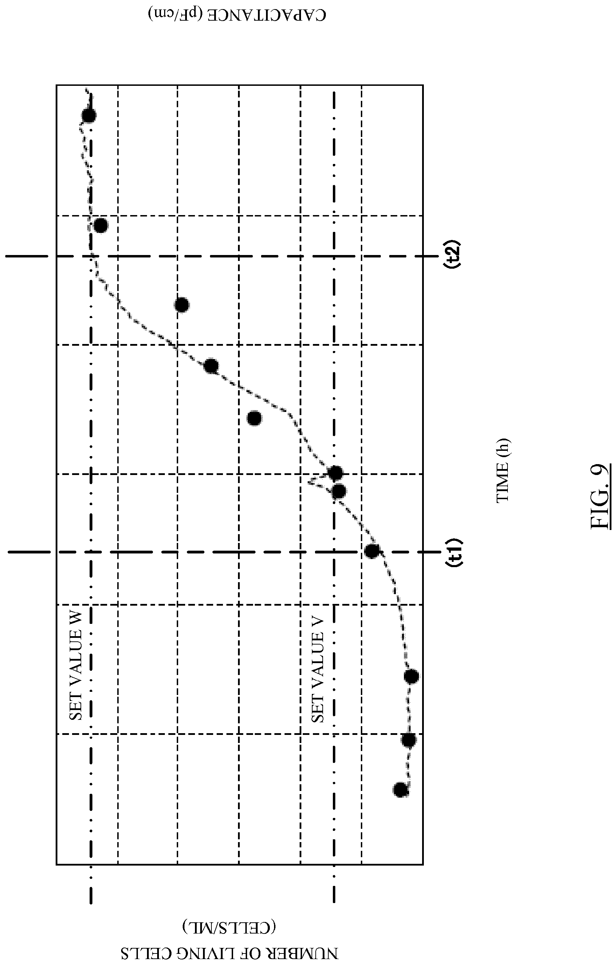

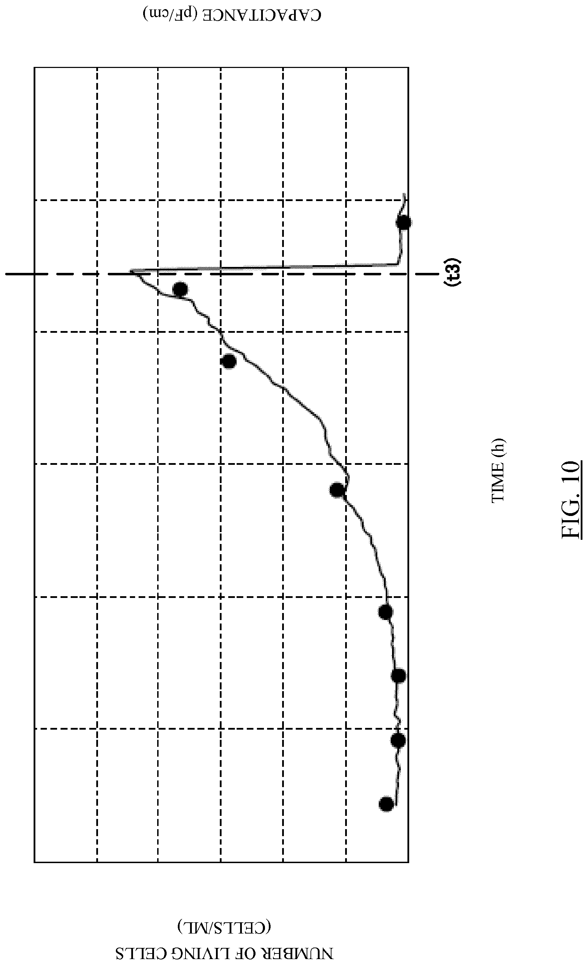

A series of operations (operations in the culture mode) in the cell culture apparatus 1 described above will be described in more detail with reference to Figs. 8 to 10. Fig. 8 is a flowchart showing an example of a part of an operation performed in the cell culture apparatus 1 according to an embodiment. Figs. 9 and 10 are views illustrating changes in capacitance and number of living cells with a lapse of time in relation to a proliferation status of cells (for example, mesenchymal stem cells) by the cell culture apparatus 1 according to an embodiment. In a series of operations described below, it is assumed that both the first threshold and the second threshold described above are set in advance as the threshold. In Figs. 9 and 10, the dotted line indicates the capacitance value (pF/cm), and the black circle indicates the number of living cells (cells/mL).

As illustrated in Fig. 8, first, cells to be cultured and culture carriers are accommodated in the culture container 10 containing a culture medium, and suspension culture is started. Thereafter, when a predetermined time has elapsed, in step (hereinafter, referred to as "ST") 500, a suspension containing at least cell complexes that are complexes of cells and culture carriers is formed or accommodated in the culture container 10 (accommodation step).

Next, in ST501, the drive device 20 agitates the suspension in the culture container 10 by a predetermined driving method (stopping for a predetermined time, intermittent driving, continuous driving, or the like) (agitation step). The agitation of the suspension by the drive device 20 may be started before ST500.

Next, in ST502, the measurement device 30 or first measurement device 300 measures a cell distribution information value (the number of living cells or a concentration of living cells) in at least a partial region or region P in the suspension (measurement step). In the measurement step according to ST502, the carrier distribution information value described in detail in modifications described below may be measured by the measurement device 30 (second measurement device 350).

Next, in ST503, the measurement device 30 (first measurement device 300) transmits the measured cell distribution information value (the number of living cells or the concentration of living cells) to the control device 40. In a case where the carrier distribution information value is measured in ST502, the measurement device 30 (second measurement device 350) may transmit the measured carrier distribution information value (the number of culture carriers or the concentration of culture carriers) to the control device 40 in ST503.

Next, in ST504, the determination unit 43 of the control device 40 determines a relationship between a change rate per predetermined unit time of the cell distribution information value and a preset threshold (for example, the first threshold and the second threshold described above) every predetermined unit time, and transmits the determination result to the output unit 44. In a case where the carrier distribution information value is measured in ST502, in ST504, the determination unit 43 of the control device 40 may determine a relationship between a change rate per predetermined unit time of the carrier distribution information value and a preset threshold (for example, a third threshold and a fourth threshold described below) every predetermined unit time, and may transmit the determination result to the output unit 44.

Next, in ST505, on the basis of the determination result of the determination unit 43, the output unit 44 of the control device 40 determines an agitation speed of the drive device 20 (agitation speed determination step).

Then, in ST506, the communication unit 41 of the control device 40 outputs the agitation speed of the drive device 20 determined by the output unit 44 to the drive device 20 as a control command to the drive device 20 (output step). Therefore, the drive device 20 outputs an agitation speed according to the control command.

Thereafter, the cell culture apparatus 1 repeats the respective operations related to ST502 to ST506 as long as the culture process of cells is continued (ST507). That is, in ST502 to ST506, the control device 40 controls driving of the drive device 20 as the culture mode. In a case where the number of living cells (concentration of living cells) reaches a target value, when the change rate in the cell distribution information value is constant for a predetermined time "0" regardless of the agitation speed of the drive device 20, or when the determination by the determination unit 43 every predetermined unit time is "abnormal" a plurality of times in a row during culture, the series of operations is ended or stopped. The "target value of the number of living cells (concentration of living cells)" described above varies depending on the type of cells to be cultured, culture scale, use, and the like, and can be set to the number of living cells (concentration of living cells) that is, for example, 5 times to 100 times, 5 times to 50 times, or 10 to 30 times the concentration of living cells at the start of the culture. In the case other than the culture mode (second mode), that is, in the bead to bead transfer mode (first mode), a specific operation of the cell culture apparatus 1 will be described below in detail.

Next, a proliferation status of cells based on the series of operations of ST500 to ST506 will be described with reference to Figs. 9 and 10. In Figs. 9 and 10, the predetermined unit time is set to 1 hour or 60 minutes. In Figs. 9 and 10, as the "change rate" described above based on the determination of the determination unit 43, a "change rate in a capacitance" is used instead of the "change rate in the cell distribution information value".

Referring to Fig. 9, in particular, since the change rate (the change rate in the capacitance) in the predetermined unit time is equal to or greater than a preset second threshold (Y described above) for times t1 to t2, the agitation speed of the drive device 20 is increased every predetermined unit time (every 1 hour) during this period. Conversely, the agitation speed of the drive device 20 is increased every predetermined unit time, such that the change rate is also increased to be equal to or greater than the second threshold. From this, it is understood that sedimentation of the cell masses, the cell complexes, and the like is suppressed in the culture container 10, and proliferation of cells (manufacture of a cell group) is efficiently executed. After the time t2, regardless of the agitation speed of the drive device 20, the change rate in the capacitance is "0" for a certain period of time. Therefore, it is understood that the proliferation of cells (manufacture of a cell group) has substantially ended (the number of living cells has reached the target value). In Fig. 9, a location where the value of the capacitance is temporarily decreased is illustrated, but this is a temporary fluctuation of the capacitance value due to the replacement of the culture medium, and does not affect the control of the agitation speed of the drive device 20.

On the other hand, referring to Fig. 10, the case illustrated in Fig. 10 is basically similar to the case illustrated in Fig. 9, but the change rate in the capacitance is rapidly decreased near a time t3, and the change rate after the time t3 is determined to be less than the first threshold (0) (abnormal) a plurality of times in a row every predetermined unit time. Therefore, in the case illustrated in Fig. 10, the control device 40 finally controls the agitation speed of the drive device 20 to be 0 so that the drive device 20 is stopped. As illustrated in Fig. 10, assuming a case where the change rate is rapidly decreased, an additional threshold may be further provided for a value at which the change rate is less than 0 (for example, -10% or the like). As described above, the cell culture apparatus 1 according to an embodiment can appropriately stop the culture when determined to be abnormal, and thus is useful in terms of time efficiency from the viewpoint of investigation of the cause determined to be abnormal, prevention of unnecessary culture progress, and the like.

### 3. Modification of cell culture apparatus 1

Next, a modification of the cell culture apparatus 1 according to an embodiment described above will be described.

### 3-1. Cell culture apparatus 1 according to Modification 1

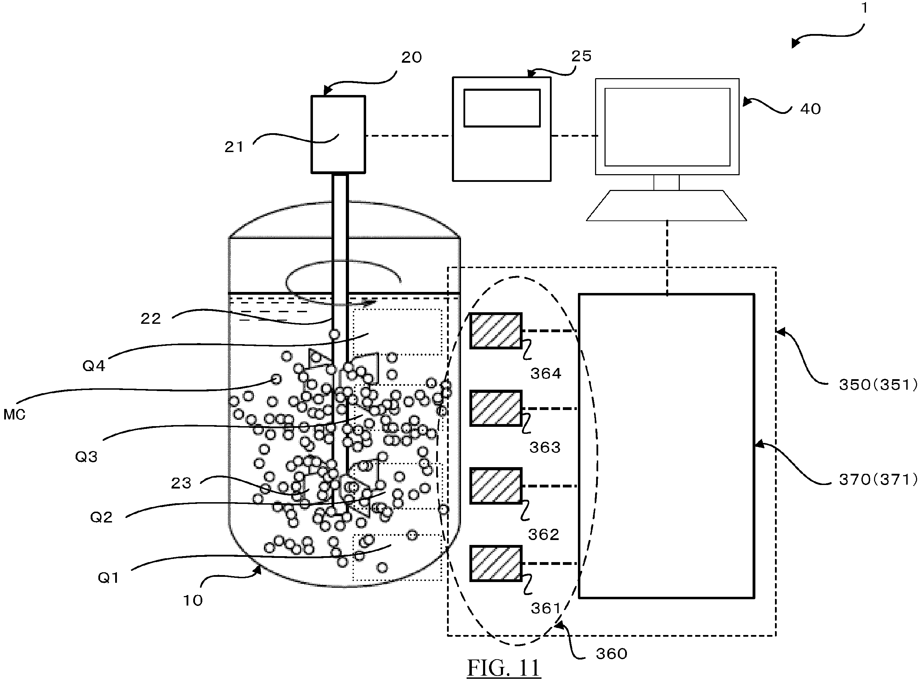

A cell culture apparatus 1 according to Modification 1 will be described with reference to Fig. 11. Fig. 11 is a schematic view schematically illustrating a configuration in a case where a second measurement device 350 is used as a measurement device 30 in a cell culture apparatus 1 according to Modification 1.

The cell culture apparatus 1 according to Modification 1 is substantially similar to the cell culture apparatus 1 according to an embodiment described above, but the second measurement device 350 is applied instead of the first measurement device 300. Therefore, in the cell culture apparatus 1 according to Modification 1, the second measurement device 350 will be described in detail as follows, and detailed descriptions of other components will be omitted.

The second measurement device 350 applied to the cell culture apparatus 1 according to Modification 1 can include an imaging unit 360 that acquires an image (may also be a moving image) by imaging culture carriers (reference numeral MC in Fig. 11) contained in a suspension in at least a partial region in a culture container 10, and a main unit 370 that measures a carrier distribution information value in the region on the basis of the captured image by the imaging unit 360. The "measurement" of the carrier distribution information value by the second measurement device 350 includes "calculation" of the carrier distribution information value on the basis of the image. As described above, when the culture carriers MC are introduced into the suspension, the cells (adherent cells) mainly proliferate on the culture carriers MC, such that cell complexes that are complexes of the cells and the culture carriers MC are formed.

As the imaging unit 360, a generally known image sensor or the like capable of acquiring a two-dimensional or three-dimensional image by continuously imaging a subject at a predetermined interval can be used. As the imaging unit 360, a large imaging unit capable of uniformly imaging the inside of the culture container 10 as viewed in a depth direction may be used. As illustrated in Fig. 11, a plurality (for example, four in Fig. 11) of small imaging units 361 to 364 may be arranged side by side in the depth direction with respect to the culture container 10, and these four imaging units 361 to 364 may be arranged at different circumferential positions (for example, the imaging unit 361 is arranged at the "12 o'clock position", the imaging unit 362 is arranged at the "3 o'clock position", the imaging unit 363 is arranged at the "6 o'clock position", and the imaging unit 364 is arranged at the "9 o'clock position") on an outer peripheral surface of the culture container 10. In some cases, only the imaging unit 361 illustrated in Fig. 11 may be provided.

The main unit 370 has a function of continuously acquiring images acquired by imaging by the imaging unit 360, recognizing the culture carriers MC included in the images, and then counting the number of culture carriers MC included in the images as the carrier distribution information value. Specifically, for example, the counting of the carrier distribution information value by the main unit 370 may be performed on the images continuously acquired by imaging by any one of the imaging units 361 to 364. Alternatively, in the counting of the carrier distribution information value by the main unit 370, for example, images acquired by imaging by at least two of the imaging units 361 to 364 may be continuously acquired, carrier distribution information values on the basis of the images acquired from each imaging unit may be calculated, and an average value thereof may be calculated as a final carrier distribution information value. The main unit 370 may have a function of counting the number of cells in the complexes of the cells and the culture carriers MC as a cell distribution information value by the imaging unit 360 (imaging units 361 to 364).

The main unit 370 may have a function of calculating the concentration of the culture carriers included in the image as the carrier distribution information value on the basis of the color or shade of the image as an alternative function of directly recognizing and counting the culture carriers MC. In such a case, the main unit 370 may learn a large number of colors or shades corresponding to various concentrations of the culture carriers in advance to store data associated with the colors or shades and the concentration of the culture carriers, and may calculate the concentration of the culture carriers included in the image acquired by the imaging unit 360 on the basis of the data.

The main unit 370 transmits the number of culture carriers MC or the concentration of the culture carriers calculated as described above to a control device 40, and the control device 40 executes processing and control similar to those in an embodiment. As described above, in a case where the number of cells in the cell complexes can be counted, the main unit 370 can transmit the number of cells to the control device 40 together with the number of culture carriers MC or instead of the number of culture carriers MC.

In Modification 1, in a case where the second measurement device 350 calculates the number of culture carriers MC (or the concentration of the culture carriers) as the carrier distribution information value, a third threshold (corresponding to the first threshold in an embodiment) with respect to the change rate in the carrier distribution information value per predetermined unit time is set. Furthermore, if necessary, a fourth threshold (corresponding to the second threshold in an embodiment) with respect to the change rate in the carrier distribution information value per predetermined unit time may be further set in advance. Therefore, the determination by a determination unit 43 in Modification 1 is executed on the basis of the change rate in the carrier distribution information value per predetermined unit time. The third threshold and the fourth threshold in the case may be the same as or different from the first threshold and the second threshold in an embodiment.

In Modification 1, in a case where the second measurement device 350 calculates both the number of culture carriers MC (or the concentration of the culture carriers) as the carrier distribution information value and the number of cells (the number of cells on the cell complexes) as the cell distribution information value, the second measurement device 350 may transmit the cell distribution information value and the carrier distribution information value to the control device 40. In this case, variations in determination by the determination unit 43 of the control device 40 in Modification 1 are increased, and for example, can be set as shown in Table 1. The cell distribution information value and the carrier distribution information value in the case of set as shown in Table 1 are acquired, for example, on the basis of the images captured by the second imaging unit 363 from the top of the third imaging unit 362 from the top in Fig. 11.

**Table 1]**

| | Change rate in cell distribution information value | Change rate in carrier distribution information value | Determination by determination unit 43 |
|---|---|---|---|
| Status 1 | Equal to or greater than second threshold | Equal to or greater than fourth threshold | Extremely normal |
| Status 2 | Equal to or greater than first threshold and less than second threshold | Equal to or greater than fourth threshold | Normal |
| Status 3 | Equal to or greater than first threshold and less than second threshold | Equal to or greater than third threshold and less than fourth threshold | Almost normal |
| Status 4 | Less than first threshold | Equal to or greater than third threshold and less than fourth threshold | Abnormal sign |
| Status 5 | Less than first threshold | Less than third threshold | Abnormal |

In a case where the second measurement device 350 calculates both the cell distribution information value and the carrier distribution information value, as shown in Table 1, for example, the agitation speed of the drive device 20 can be set in advance in accordance with each determination regarding a status 1 to a status 5, such that the agitation speed of the drive device 20 can be finely controlled. Therefore, sedimentation of the cell complexes and the like in the culture container 10 can be efficiently controlled, and the yield and time efficiency of the manufacture of the cell group can be further improved.

### 3-2. Cell culture apparatus 1 according to Modification 2

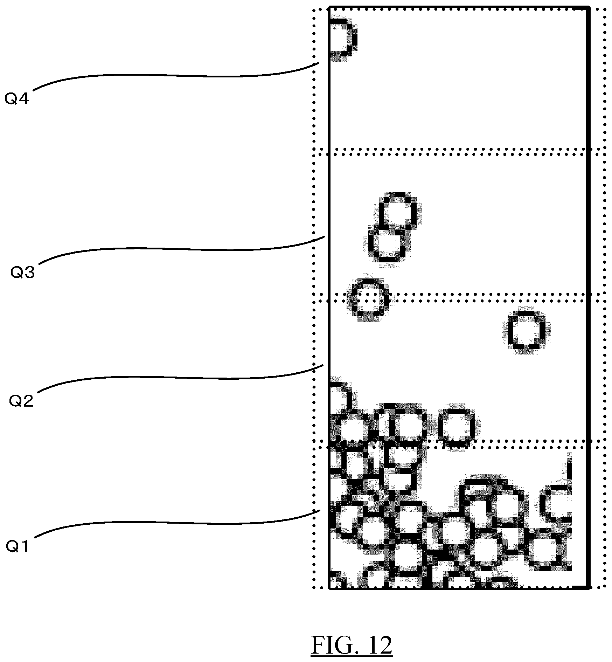

Next, a cell culture apparatus 1 according to Modification 2 will be described with reference to Fig. 12. Fig. 12 is a view illustrating an example of an image captured by an imaging unit 360 in the cell culture apparatus 1 according to Modification 2.

The cell culture apparatus 1 according to Modification 2 is physically the same as the cell culture apparatus 1 according to Modification 1 illustrated in Fig. 11 described above, but is different from that of Modification 1 in a usage mode of an image acquired by imaging by an imaging unit 360 by a main unit. That is, the functions of the main units are slightly different between Modification 1 and Modification 2. Therefore, for convenience, a measurement device 30 used in Modification 2 will be referred to as a second measurement device 351, and the main unit of the second measurement device 351 will be referred to as a main unit 371.

In the second measurement device 351 according to Modification 2, the same imaging unit as the imaging unit 360 according to Modification 1 can be used as the imaging unit 360. Then, the second measurement device 351 measures each of carrier distribution information values in a plurality of regions in a suspension in a culture container 10, and calculates a carrier distribution information difference value by a comparison operation between a first carrier distribution information value in a first region among the plurality of regions and a second carrier distribution information value in a second region among the plurality of regions. Then, an agitation speed of a drive device 20 is controlled on the basis of a relationship between the carrier distribution information difference value and at least one or more preset thresholds for the carrier distribution information difference value.

The first carrier distribution information value means a carrier distribution information value (specifically, the number of culture carriers or a concentration of the culture carriers) in the first region, and the second carrier distribution information value means a carrier distribution information value in the second region.

More specifically, as illustrated in Figs. 11 and 12, as the imaging unit 360 according to Modification 2, a plurality (for example, four in Fig. 11) of small imaging units may be used, or one large imaging unit 360 may be used. For example, as illustrated in Figs. 11 and 12, in a case where the imaging unit 360 is four small imaging units 361 to 364 (or at least two imaging units thereof), these imaging units 361 to 364 image the corresponding regions Q1 to Q4 (or at least two regions among the regions Q1 to Q4) of the suspension.

Then, each of the imaging units 361 to 364 transmits an image captured and acquired in each region to the main unit 371. Therefore, the main unit 371 measures each of carrier distribution information values in the regions Q1 to Q4 (or at least two regions among these regions), and transmits each of the measured carrier distribution information values to a control device 40.

The control device 40 that acquires each of the carrier distribution information values in the regions Q1 to Q4 (or at least two regions among these regions) from the main unit 371 calculates, for example, a carrier distribution information difference value by a comparison operation between a carrier distribution information value (first carrier distribution information value) in the region Q3 (corresponding to the first region) and a carrier distribution information value (second carrier distribution information value) in the region Q1 (corresponding to the second region). The carrier distribution information difference value is obtained, for example, by subtracting the second carrier distribution information value from the first carrier distribution information value.

A determination unit 43 of the control device 40 monitors the calculated carrier distribution information difference value, and executes determination such as "normal" or "abnormal" as in an embodiment on the basis of the relationship between the carrier distribution information difference value and a preset threshold.

For example, when the value obtained by subtracting the carrier distribution information value (second carrier distribution information value) in the region Q1 from the carrier distribution information value (first carrier distribution information value) in the region Q3 is substantially 0, the culture carriers are considered to be uniformly dispersed in the suspension and thus can be determined as "normal", and when the subtracted value is significantly less than 0, the culture carriers are considered to be precipitated and the like and thus can be determined as "abnormal". Therefore, sedimentation of cell masses, cell complexes, and the like in the culture container 10 can be efficiently controlled, and the yield and time efficiency of manufacture of a cell group can be further improved.

### 3-3. Cell culture apparatus 1 according to Modification 3

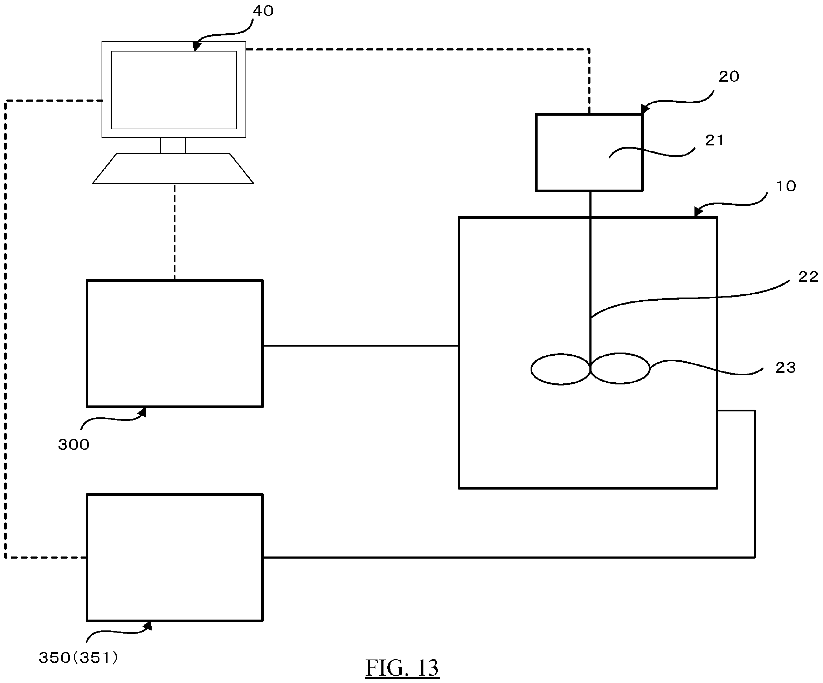

Next, a cell culture apparatus 1 according to Modification 3 will be described with reference to Fig. 13. Fig. 13 is a schematic view schematically illustrating a configuration of the cell culture apparatus 1 according to Modification 3.

In the cell culture apparatus 1 according to Modification 3, both a first measurement device 300 applied to the cell culture apparatus 1 according to an embodiment and a second measurement device 350 (and a second measurement device 351) applied to the cell culture apparatus 1 according to Modification 1 (and Modification 2) are applied. Therefore, a control device 40 according to Modification 3 can receive a large number of values related to a cell distribution information value and a carrier distribution information value (in some cases, only a cell distribution information value may be used) from the first measurement device 300 and the second measurement device 350 (and the second measurement device 351). Therefore, variations in determination of a determination unit 43 of the control device 40 can be maximized. As a result, an agitation speed of a drive device 20 can be more finely controlled. Therefore, sedimentation of cell masses, cell complexes, and the like in a culture container 10 can be more efficiently controlled, and the yield and time efficiency of manufacture of a cell group can be further improved.

### 4. Function of cell culture apparatus 1 related to bead to bead transfer

Next, details of the function of the cell culture apparatus 1 related to the bead to bead transfer will be described. The cell culture in the present disclosure is basically managed in two modes of the culture mode (second mode) described above and a bead to bead transfer mode (first mode) described below.

In a culture process of cells according to the present disclosure, in addition to the culture carriers introduced into the suspension at the start of the culture process (in the present disclosure, for convenience, also referred to as "initial culture carriers"), new culture carriers (in the present disclosure, for convenience, also referred to as "additional culture carriers") are added into the suspension during the culture process (in the middle of the culture process). Therefore, the cells present on (adhered to) the initial culture carriers can be transferred (migrated) onto the additional culture carriers to improve the yield (manufacture) of aggregates (a cell group) of cultured cells. It is important to efficiently advance the transfer of the cells present on the initial culture carriers onto the additional culture carriers (in the present disclosure, also referred to as "bead to bead transfer") from the viewpoint of improving the yield of the cell group and time efficiency. As the additional culture carriers, fresh culture carriers (unused culture carriers) are preferably used.

When the initial culture carriers are added into the culture container 10 (in the suspension), a suspension containing a predetermined amount of the initial culture carriers may be prepared on the basis of the shape, size, surface area, and the like of the culture carrier using a culture medium and the like. A concentration of the initial culture carriers in the corresponding suspension can be, for example, 0.01 to 100 g/L, 0.5 to 50 g/L, or 1 to 20 g/L.

In the present disclosure, the bead to bead transfer mode means a mode of the control device 40 in order to add additional culture carriers to the suspension during the culture process of the cells to reliably transfer the cells present on the initial culture carriers onto the additional culture carriers. The control device 40 in the cell culture apparatus 1 described above can execute various control, management, and the like as the bead to bead transfer mode in order to efficiently advance the bead to bead transfer.

First, the control device 40 can determine a time point at which the additional culture carriers are introduced into the culture container 10 in the culture process of the cells.

As described above, the communication unit 41 of the control device 40 receives the cell distribution information value from the measurement device 30 (may be any one of the first measurement device 300, the second measurement device 350, and the second measurement device 351 described above) and transmits the received cell distribution information value to the storage unit 42. The determination unit 43 of the control device 40 can determine a time point at which the cell distribution information value reaches a predetermined set value as a time point at which the additional culture carriers are added into the culture container 10. The predetermined set value may be stored in the storage unit 42. Therefore, the determination unit 43 can determine a time point at which the cell distribution information value reaches the set value by reading the set value from the storage unit 42 and comparing the cell distribution information value received via the communication unit 41 and the set value read from the storage unit 42. Accordingly, it is possible to add the additional culture carriers to the culture container 10 at an appropriate timing. When the additional culture carriers are added into the culture container 10 (in the suspension), a suspension containing a predetermined amount of the additional culture carriers is prepared on the basis of the shape, size, surface area, and the like of the additional culture carrier using a culture medium and the like. A concentration of the additional culture carriers in the corresponding suspension can be, for example, 0.01 to 100 g/L, 0.5 to 50 g/L, or 1 to 20 g/L. When the suspension containing the additional culture carriers are added into the culture container 10, a volume of the suspension already accommodated in the culture container 10 or a volume of the suspension containing the additional culture carriers may be appropriately adjusted in consideration of a volume of the culture container 10.

The predetermined set value for the cell distribution information value may be appropriately set from an empirical rule. For example, a set value V or a set value W may be set as the set value as illustrated in Fig. 9. Therefore, the determination unit 43 can determine a time point at which the cell distribution information value reaches a set value A or a set value B as the time point at which the additional culture carriers are added into the culture container 10.

Next, as for driving of the drive device 20, the control device 40 can control driving of the drive device 20 in accordance with the bead to bead transfer on the basis of at least one of the cell distribution information value and the carrier distribution information value measured by the measurement device 30. At this time, since there are various variations in the drive control of the drive device 20 by the control device 40 in accordance with the bead to bead transfer, each of the variations will be described below. In the bead to bead transfer mode (first mode), the time point at which the additional culture carriers are added into the culture container 10 is defined as a substantial start time, and the bead to bead transfer mode is ended when a predetermined condition is satisfied. The determination as to whether or not the predetermined condition is satisfied will be described in each variation. It should be understood that the cell culture basically is advanced in the culture mode (second mode) before the start of the bead to bead transfer mode and after the end of the bead to bead transfer mode unless the cell culture is stopped for some reason.

### 4-1. First variation

First, a first variation of the drive control of the drive device 20 by the control device 40 in accordance with the bead to bead transfer will be described.

When the additional culture carriers are added into the culture container 10 as described above, the control device 40 in the first variation can control driving of the drive device 20 by monitoring the progress of the bead to bead transfer of the cells on the cell complexes onto the additional culture carriers on the basis of the first change value calculated by comparing the cell distribution information value at the first time point after the addition of the additional culture carriers and the cell distribution information value at the second time point after the first time point.

Specifically, the communication unit 41 of the control device 40 can continuously acquire the number of living cells (or the concentration of living cells) as a cell distribution information value in a certain region (for example, the region P in Fig. 2) in the suspension from the first measurement device 300 or the second measurement device 350. The determination unit 43 of the control device 40 can grasp a change in the number of living cells (the concentration of living cells) in a certain region from the first time point to the second time point by a comparison operation (for example, subtraction) between the number of living cells (the concentration of living cells) in a certain region at the first time point after the addition of the additional culture carriers and the number of living cells (the concentration of living cells) in a certain region at the second time point after the first time point.

The first time point may be any time point immediately after the additional culture carriers are added, and may be set automatically or manually. The second time point may be any time point after the first time point, and for example, a plurality of time points such as "x₁ minutes later", "x₂ minutes later", and "x₃ minutes later" later than the first time point (the plurality of time points may be at equal time intervals or at different time intervals) are collectively referred to as the second time point. It can be understood that the first time point and the second time point are any time points in the bead to bead transfer mode. It should be understood that at least the driving of the drive device 20 is not stopped and a predetermined agitation speed is output at the first time point and the second time point.

As shown in the region P in Fig. 2, the certain region may be the central portion of the culture container 10 as viewed in the depth direction, or may be the vicinity of the bottom surface of the culture container 10.

At the time point at which the additional culture carriers are added into the suspension, the culture process of the cells is advanced for a certain period of time, and thus the cells are usually proliferated on the initial culture carriers to a certain degree. That is, the cell complexes of the initial culture carriers and the cells are formed. When the additional culture carriers are added in this state, the cell complexes of the initial culture carriers and the cells and the additional culture carriers are mixed in the suspension. A specific gravity of the cell complexes of the initial culture carriers and the cells is significantly different from a specific gravity of the additional culture carriers. Therefore, when the suspension is agitated in a state where the cell complexes of the initial culture carriers and the cells and the additional culture carriers are mixed, in general, the cell complexes of the initial culture carriers and the cells settle in the vicinity of the bottom surface of the culture container 10, and the additional culture carriers float in the central portion of the culture container 10 as viewed in the depth direction. However, when the transfer of the cells on the initial culture carriers onto the additional culture carriers, that is, the bead to bead transfer is advanced or completed, regardless of the initial culture carriers or the additional culture carriers, the cell complexes of the cells and the culture carriers are uniform (the specific gravity is uniform) to a certain degree. In other words, the cells adhere substantially uniformly to the initial culture carriers and the additional culture carriers. As described above, it is important to allow the cells to adhere substantially uniformly to all the culture carriers and then advance the culture process in a normal culture mode so as to improve the yield (manufacture) of the cell group.

In the first variation, the determination unit 43 of the control device 40 determines whether to drive the drive device 20 in the bead to bead transfer mode (first mode) or the culture mode (second mode) on the basis of a relationship between the first change value and a preset first predetermined value (stored in advance in the storage unit 42). For example, the determination unit 43 determines to drive the drive device 20 in the bead to bead transfer mode (first mode) until the first change value reaches the first predetermined value. On the other hand, when the first change value reaches the first predetermined value, the determination unit 43 presumes that the bead to bead transfer is completed and determines to drive the drive device 20 in the culture mode (second mode).

The first predetermined value can be appropriately determined and changed. In a case where the "certain region" is, for example, the vicinity of the bottom surface of the culture container 10, the cell complexes of the initial culture carriers and the cells settle in the vicinity of the bottom surface at the first time point, and thus the cell distribution information value in the vicinity of the bottom surface at the first time point becomes a relatively large value. Thereafter, when the bead to bead transfer is advanced, regardless of the initial culture carriers or the additional culture carriers, the cell complexes of the cells and the culture carriers (or the specific gravity thereof) are uniform to a certain degree, and as a result, the cell distribution information value in the vicinity of the bottom surface is gradually decreased or changed. Therefore, the first predetermined value in this case is set in advance with respect to the degree of decrease in the cell distribution information value.

In a case where the "certain region" is, for example, the central portion of the culture container 10 as viewed in the depth direction, the cell complexes of the initial culture carriers and the cells settle in the vicinity of the bottom surface at the first time point, and thus the cell distribution information value in the central portion at the first time point becomes a relatively small value. Thereafter, when the bead to bead transfer is advanced, the cell distribution information value at the central portion is gradually increased or changed. Therefore, the first predetermined value in this case is set in advance with respect to the degree of increase in the cell distribution information value.

Meanwhile, in a case where the determination unit 43 determines that the bead to bead transfer is completed and determines to drive the drive device 20 in the culture mode with the fact that the first change value reaches the first predetermined value as a trigger, the output unit 44 outputs the agitation speed as described in detail above on the basis of each determination of the determination unit 43 in the culture mode to drive the drive device 20.

On the other hand, when the determination unit 43 determines that the first change value does not reach the first predetermined value and determines to drive the drive device 20 in the bead to bead transfer mode (first mode), the output unit 44 controls the drive device 20 on the basis of the agitation speed corresponding to the bead to bead transfer mode.

In a case where the drive device 20 is driven in the bead to bead transfer mode, specifically, the output unit 44 intermittently drives the drive device 20. The intermittent driving means that the agitation of the drive device 20 is executed or the execution of the agitation is stopped every predetermined time. More specifically, the intermittent driving means alternately executing the agitation of the drive device 20 for a predetermined time and stopping the agitation of the drive device 20 for a predetermined time. The alternative executing may mean repeating a combination of executing the agitation and stopping the agitation two or more times ("executes agitation" and "stops agitation" subsequent thereto are counted as one combination). The time corresponding to the execution of the agitation and the time corresponding to the stop of the agitation may be the same as or different from each other. In the repetition of two or more times, the times corresponding to the execution of the agitation between the number of times may be always the same as or different from each other. Similarly, the times corresponding to the stop of the agitation between the number of times may be always the same as or different from each other. The time for stopping the agitation is to be understood as the time for allowing the suspension to stand in the culture container 10 to promote the bead to bead transfer of the cells. Any one of the first time point and the second time point is a time corresponding to the execution of the agitation. The agitation speed of the drive device 20 in the case of "executing the agitation" in the intermittent driving may be the same as or different from the specific agitation speed of the drive device 20 described above.

The time corresponding to the execution of the agitation is, for example, 0.5 to 60 minutes, and can be preferably 1 to 20 minutes and more preferably 3 to 10 minutes. The time corresponding to the stop of the agitation is, for example, 0.1 to 10 hours, and can be preferably 0.5 to 6 hours and more preferably 1 to 3 hours. Examples of a combination of "the execution of the agitation" and then "the stop of the agitation" include ""agitation for 0.5 to 60 minutes" and then "stop of the agitation for 0.1 to 10 hoursʺʺ, and ""agitation for 1 to 20 minutes" and then "stop of the agitation for 0.5 to 6 hoursʺʺ is preferable, and ""agitation for 3 to 10 minutes" and then "stop of the agitation for 1 to 3 hoursʺʺ is more preferable.

The time for performing the intermittent driving (total time for performing ""the execution of the agitation" and then "the stop of the agitationʺʺ two or more times) is, for example, 1 to 80 hours, preferably 10 to 40 hours, and more preferably 20 to 30 hours.

As described above, the intermittent driving is executed at the start of the culture process in which the initial culture carriers are added into the culture container 10 (into the suspension) (the intermittent driving in this case is also referred to as "initial intermittent driving" in the present disclosure for convenience). The initial intermittent driving is executed for the purpose of promoting adhesion of the cells to the initial culture carriers. After the initial intermittent driving is executed, the drive device 20 is driven (continuously driven) in the culture mode described above (further, thereafter, the intermittent driving based on the bead to bead transfer mode is executed in response to the addition of the additional culture carriers). The continuous driving in the culture mode means that the agitation is continuously performed for a predetermined time. The time for the agitation (the case where the culture is stopped by the determination of "abnormal" described above is excluded) is, for example, 1 to 14 days, preferably 2 to 10 days, and more preferably 3 to 7 days. In the cell culture, the agitation may be temporarily stopped, for example, due to addition of a culture medium to the suspension, replacement of the culture medium, and the like. In the present disclosure, the temporary stop means that the agitation is temporarily stopped for a treatment such as addition of the culture medium to the suspension or the replacement of the culture medium, rather than simply allowing the suspension to stand. The time for the agitation may not be the sum of the time for the agitation before the temporary stop and the time for the agitation after the temporary stop. That is, the measurement may be performed by discriminating the time for the agitation by the stop of the agitation.

The intermittent driving described above and the continuous driving according to the culture mode described above may be performed continuously, or may include no agitation for a predetermined time between the intermittent driving and the continuous driving. Similarly, the initial intermittent driving described above and the continuous driving according to the culture mode described above may be performed continuously, or may include no agitation for a predetermined time between the initial intermittent driving and the continuous driving. The predetermined time is, for example, 0.1 to 24 hours, preferably 0.5 to 5 times, and more preferably 1 to 2 hours. No agitation may be allowing the suspension to stand. At the start of the culture process, a combination of initial intermittent driving and continuous driving may be repeated.

Returning to the details of the first variation, when the timing of adding the additional culture carriers is automatically determined as described above, the determination unit 43 may simultaneously determine to start driving the drive device 20 in the bead to bead transfer mode at the time of this determination. Accordingly, the output unit 44 can drive the drive device 20 in the bead to bead transfer mode at the same time (substantially at the same time) when the additional culture carriers are added into the culture container 10.

As described above, the driving of the drive device 20 in accordance with the bead to bead transfer is controlled, such that the bead to bead transfer of the cells can be advanced efficiently. As a result, it is possible to culture cells (manufacture a cell group) with a high yield and time efficiency.

### 4-2. Second variation

Next, a second variation of the drive control of the drive device 20 by the control device 40 in accordance with the bead to bead transfer will be described.

The second variation is different from the first variation in that, in the first variation, the cell distribution information value in a certain region is used for the determination of the determination unit 43, but in the second variation, the carrier distribution information value in the certain region is used for the determination of the determination unit 43 instead of the cell distribution information value in the certain region. In other respects, the first variation and the second variation are basically the same.

That is, when the additional culture carriers are added into the culture container 10 as described above, the control device 40 in the second variation can control driving of the drive device 20 by monitoring the progress of the bead to bead transfer of the cells on the cell complexes onto the additional culture carriers on the basis of the second change value calculated by comparing the carrier distribution information value at the first time point after the addition of the additional culture carriers and the carrier distribution information value at the second time point after the first time point.

Specifically, as described above, the communication unit 41 of the control device 40 can continuously acquire the number of culture carriers (or the concentration of the culture carriers) as a carrier distribution information value in a certain region (for example, the region P in Fig. 2) in the suspension from the first measurement device 300 or the second measurement device 350. The determination unit 43 of the control device 40 can grasp a change in the number of culture carriers (the concentration of the culture carriers) in a certain region from the first time point to the second time point by a comparison operation (for example, subtraction) between the number of culture carriers (the concentration of the culture carriers) in a certain region at the first time point after the addition of the additional culture carriers and the number of culture carriers (the concentration of the culture carriers) in a certain region at the second time point after the first time point. The "number of culture carriers" used here means a total number of initial culture carriers and additional culture carriers. The "concentration of the culture carriers" means a total concentration of a concentration of initial culture carriers and a concentration of additional culture carriers.

The "certain region", the "first time point", the "second time point", and the like described in detail in the first variation are similarly applied to the second variation.

In the second variation, the determination unit 43 of the control device 40 determines whether to drive the drive device 20 in the bead to bead transfer mode (first mode) or the culture mode (second mode) on the basis of a relationship between the second change value and a preset second predetermined value (stored in advance in the storage unit 42). For example, the determination unit 43 determines to drive the drive device 20 in the bead to bead transfer mode (first mode) until the second change value reaches the second predetermined value. On the other hand, when the second change value reaches the second predetermined value, the determination unit 43 presumes that the bead to bead transfer is completed and determines to drive the drive device 20 in the culture mode (second mode).

The second predetermined value can be appropriately determined and changed similarly to the first predetermined value in the first variation.

Also in the second variation, similarly to the first variation, in a case where the determination unit 43 determines that the bead to bead transfer is completed and determines to drive the drive device 20 in the culture mode with the fact that the second change value reaches the second predetermined value as a trigger, the output unit 44 outputs the agitation speed as described in detail above on the basis of each determination of the determination unit 43 in the culture mode to drive the drive device 20.

On the other hand, when the determination unit 43 determines that the second change value does not reach the second predetermined value and determines to drive the drive device 20 in the bead to bead transfer mode (first mode), the output unit 44 controls the drive device 20 on the basis of the agitation speed corresponding to the bead to bead transfer mode. The drive control of the drive device 20 corresponding to the bead to bead transfer mode is as described above.

Similarly to the first variation, in the second variation, when the timing of adding the additional culture carriers is automatically determined as described above, the determination unit 43 may simultaneously determine to start driving the drive device 20 in the bead to bead transfer mode at the time of this determination. Accordingly, the output unit 44 can drive the drive device 20 in the bead to bead transfer mode at the same time (substantially at the same time) when the additional culture carriers are added into the culture container 10.

As described above, similarly to the first variation, in the second variation, the driving of the drive device 20 in accordance with the bead to bead transfer is controlled, such that the bead to bead transfer of the cells can be advanced efficiently. As a result, it is possible to culture cells (manufacture a cell group) with a high yield and time efficiency.

The drive control of the drive device 20 by the control device 40 in accordance with the bead to bead transfer may be a combination of the first variation and the second variation. The determination of the determination unit 43 in this case is executed on the basis of both the cell distribution information value and the carrier distribution information value.

### 4-3. Third variation

A third variation of the drive control of the drive device 20 by the control device 40 in accordance with the bead to bead transfer will be described.

When the additional culture carriers are added into the culture container 10 as described above, the control device 40 in the third variation can control the driving of the drive device 20 by monitoring the progress of the bead to bead transfer of the cells on the cell complexes onto the additional culture carriers on the basis of the carrier distribution information difference value calculated by a comparison operation between the first carrier distribution information value in the first region in at least the partial region (among the plurality of regions) in the culture container 10 (in the suspension) and the second carrier distribution information value in the second region in at least the partial region after the addition of the additional culture carriers.

Specifically, as described above, for example, the control device 40 can continuously acquire the number of culture carriers, or concentration of the culture carriers as the first carrier distribution information value in the first region and the number of culture carriers, or concentration of the culture carriers as the second carrier distribution information value in the second region in the suspension in the culture container 10 from the second measurement device 351 according to Modification 2 described above. Therefore, as described in Modification 2, the determination unit 43 of the control device 40 can calculate a carrier distribution information difference value on the basis of the first carrier distribution information value and the second carrier distribution information value acquired from the second measurement device 351. It can be said that the carrier distribution information difference value represents a difference between the number of culture carriers, or concentration of the culture carriers in the first region and the number of culture carriers, or concentration of the culture carriers in the second region at a certain time point.

The first region in the third variation can be, for example, the region Q3 (the region imaged by the imaging unit 363) illustrated in Fig. 12 described in Modification 2. The second region in the third variation can be, for example, the region Q1 (a region imaged by the imaging unit 361) illustrated in Fig. 12 described in Modification 2. Therefore, a difference in the number of culture carriers (concentration of the culture carriers) between the central portion and the vicinity of the bottom surface in the culture container 10 can be monitored by calculating a carrier distribution information difference value by a comparison operation (for example, subtraction) between the first carrier distribution information value in the region Q3 corresponding to the central portion of the culture container 10 as viewed in the depth direction and the second carrier distribution information value in the region Q1 corresponding to the vicinity of the bottom surface of the culture container 10.

As described in detail in the first variation, immediately after the additional culture carriers are added, when the cell complexes of the initial culture carriers and the cells and the additional culture carriers are mixed, and the suspension is agitated in this state, in general, the cell complexes of the initial culture carriers and the cells have a high specific gravity and thus settle in the vicinity of the bottom surface of the culture container 10, and the additional culture carriers float in the central portion of the culture container 10 as viewed in the depth direction. Therefore, (the absolute value of) the carrier distribution information difference value immediately after the additional culture carriers are added is a large value.

However, as described in the first variation, when the transfer of the cells on the initial culture carriers onto the additional culture carriers, that is, the bead to bead transfer is advanced or completed, regardless of the initial culture carriers or the additional culture carriers, the cell complexes of the cells and the culture carriers are uniform (the specific gravity is uniform) to a certain degree. Therefore, as the bead to bead transfer is advanced, (the absolute value of) the carrier distribution information difference value is gradually decreased and finally becomes a value close to 0.

In the third variation, the determination unit 43 of the control device 40 determines whether to drive the drive device 20 in the bead to bead transfer mode (first mode) or the culture mode (second mode) on the basis of a relationship between the carrier distribution information difference value and the preset predetermined value (stored in the storage unit 42 in advance) by monitoring the change in the carrier distribution information difference value. For example, the determination unit 43 determines to drive the drive device 20 in the bead to bead transfer mode (first mode) until the carrier distribution information difference value reaches the preset predetermined value. On the other hand, when the carrier distribution information difference value reaches the preset predetermined value, the determination unit 43 presumes that the bead to bead transfer is completed and determines to drive the drive device 20 in the culture mode (second mode). The specific drive of the drive device 20 in the bead to bead transfer mode and the specific drive of the drive device 20 in the culture mode are as described above.

The preset predetermined value for the carrier distribution information difference value can be appropriately set and changed. For example, as described above, when the bead to bead transfer is advanced, the carrier distribution information difference value is gradually decreased and becomes a value close to 0, and thus the predetermined value can be set to, for example, 0 or substantially 0.

In the third variation, the timing at which the determination unit 43 executes the determination as described above is preferably at least a case where the driving of the drive device 20 is executed, and specifically, a case where the agitation of the drive device 20 is executed in a case where the drive device 20 is intermittently driven in the bead to bead transfer mode.

Similarly to the case of the first variation, when the timing of adding the additional culture carriers is automatically determined as described above, the determination unit 43 in the case of the third variation may also simultaneously determine to start driving the drive device 20 in the bead to bead transfer mode at the time of this determination. Accordingly, the output unit 44 can drive the drive device 20 in the bead to bead transfer mode substantially at the same time when the additional culture carriers are added into the culture container 10.

As described above, similarly to the first variation, in the third variation, the driving of the drive device 20 in accordance with the bead to bead transfer is controlled, such that the bead to bead transfer of the cells can be advanced efficiently. As a result, it is possible to culture cells (manufacture a cell group) with a high yield and time efficiency.

### 4-4. Fourth variation

A fourth variation of the drive control of the drive device 20 by the control device 40 in accordance with the bead to bead transfer will be described.

The fourth variation is different from the third variation in that, in the third variation, the drive control of the drive device 20 is executed using the carrier distribution information difference value calculated by a comparison operation between the carrier distribution information value in the first region and the carrier distribution information value in the second region, and in the fourth variation, the drive control of the drive device 20 is executed using the cell distribution information difference value calculated by a comparison operation between the cell distribution information value in the first region and the cell distribution information value in the second region. In other respects, the third variation and the fourth variation are generally the same.

That is, when the additional culture carriers are added into the culture container 10 as described above, the control device 40 in the fourth variation can control the driving of the drive device 20 by monitoring the progress of the bead to bead transfer of the cells on the cell complexes onto the additional culture carriers on the basis of the cell distribution information difference value calculated by a comparison operation between the first cell distribution information value in the first region in at least the partial region (among the plurality of regions) in the culture container 10 (in the suspension) and the second cell distribution information value in the second region in at least the partial region after the addition of the additional culture carriers.

Specifically, as described above, for example, the control device 40 can continuously acquire the number of living cells (concentration of living cells) as the first cell distribution information value in the first region and the number of living cells (concentration of living cells) as the second cell distribution information value in the second region in the suspension in the culture container 10 from the first measurement device 300 or the second measurement device 351 according to Modification 2 described above. Therefore, the determination unit 43 of the control device 40 can calculate a cell distribution information difference value on the basis of the first cell distribution information value and the second cell distribution information value acquired from the first measurement device 300 or the second measurement device 351. It can be said that the cell distribution information difference value represents a difference between the number of living cells (concentration of living cells) in the first region and the number of living cells (concentration of living cells) in the second region at a certain time point.

The first region in the fourth variation can be, for example, the region Q3 (the region imaged by the imaging unit 363) illustrated in Fig. 12 described in Modification 2 similarly to the third variation. The second region in the fourth variation can be, for example, the region Q1 (a region imaged by the imaging unit 361) illustrated in Fig. 12 described in Modification 2. In the fourth variation, in a case where the first cell distribution information value and the second cell distribution information value are acquired from the first measurement device 300, the electrode 310 and the capacitance measurement unit 320 in the first measurement device 300 may be disposed in the region Q1 and the region Q3, respectively.

Therefore, a difference in the number of living cells (concentration of living cells) between the central portion and the vicinity of the bottom surface in the culture container 10 can be monitored by calculating a cell distribution information difference value by a comparison operation (for example, subtraction) between the first cell distribution information value in the region Q3 corresponding to the central portion of the culture container 10 as viewed in the depth direction and the second cell distribution information value in the region Q1 corresponding to the vicinity of the bottom surface of the culture container 10.

As described in detail in the first variation, immediately after the additional culture carriers are added, when the cell complexes of the initial culture carriers and the cells and the additional culture carriers are mixed, and the suspension is agitated in this state, in general, the cell complexes of the initial culture carriers and the cells have a high specific gravity and thus settle in the vicinity of the bottom surface of the culture container 10, and the additional culture carriers float in the central portion of the culture container 10 as viewed in the depth direction. Therefore, (the absolute value of) the cell distribution information difference value immediately after the additional culture carriers are added is a large value.

However, as described in the first variation, when the transfer of the cells on the initial culture carriers onto the additional culture carriers, that is, the bead to bead transfer is advanced or completed, regardless of the initial culture carriers or the additional culture carriers, the cell complexes of the cells and the culture carriers are uniform (the specific gravity is uniform) to a certain degree. Therefore, as the bead to bead transfer is advanced, (the absolute value of) the cell distribution information difference value is gradually decreased and finally becomes a value close to 0.

In the fourth variation, the determination unit 43 of the control device 40 determines whether to drive the drive device 20 in the bead to bead transfer mode (first mode) or the culture mode (second mode) on the basis of a relationship between the cell distribution information difference value and the preset predetermined value (stored in the storage unit 42 in advance) by monitoring the change in the cell distribution information difference value. For example, the determination unit 43 determines to drive the drive device 20 in the bead to bead transfer mode (first mode) until the cell distribution information difference value reaches the preset predetermined value. On the other hand, when the cell distribution information difference value reaches the preset predetermined value, the determination unit 43 presumes that the bead to bead transfer is completed and determines to drive the drive device 20 in the culture mode (second mode). The specific drive of the drive device 20 in the bead to bead transfer mode and the specific drive of the drive device 20 in the culture mode are as described above.

The preset predetermined value for the cell distribution information difference value can be appropriately set and changed similarly to the predetermined value in the third variation. For example, as described above, when the bead to bead transfer is advanced, the cell distribution information difference value is gradually decreased and becomes a value close to 0, and thus the predetermined value can be set to, for example, 0 or substantially 0.

In the fourth variation, similarly to the third variation, the timing at which the determination unit 43 executes the determination as described above is preferably at least a case where the driving of the drive device 20 is executed, specifically, a case where the agitation of the drive device 20 is executed in a case where the drive device 20 is intermittently driven in the bead to bead transfer mode.

Similarly to the case of the first variation, when the timing of adding the additional culture carriers is automatically determined as described above, the determination unit 43 in the case of the fourth variation may also simultaneously determine to start driving the drive device 20 in the bead to bead transfer mode at the time of this determination. Accordingly, the output unit 44 can drive the drive device 20 in the bead to bead transfer mode at the same time (substantially at the same time) when the additional culture carriers are added into the culture container 10.

As described above, similarly to the third variation, in the fourth variation, the driving of the drive device 20 in accordance with the bead to bead transfer is controlled, such that the bead to bead transfer of the cells can be advanced efficiently. As a result, it is possible to culture cells (manufacture a cell group) with a high yield and time efficiency.

As described above, each of the first variation to the fourth variation has been described. However, the drive control of the drive device 20 by the control device 40 in accordance with the bead to bead transfer may be executed by only one of the first variation to the fourth variation, or may be executed by combining two or more of these variations.

### 5. Operation of cell culture apparatus 1 in bead to bead transfer mode

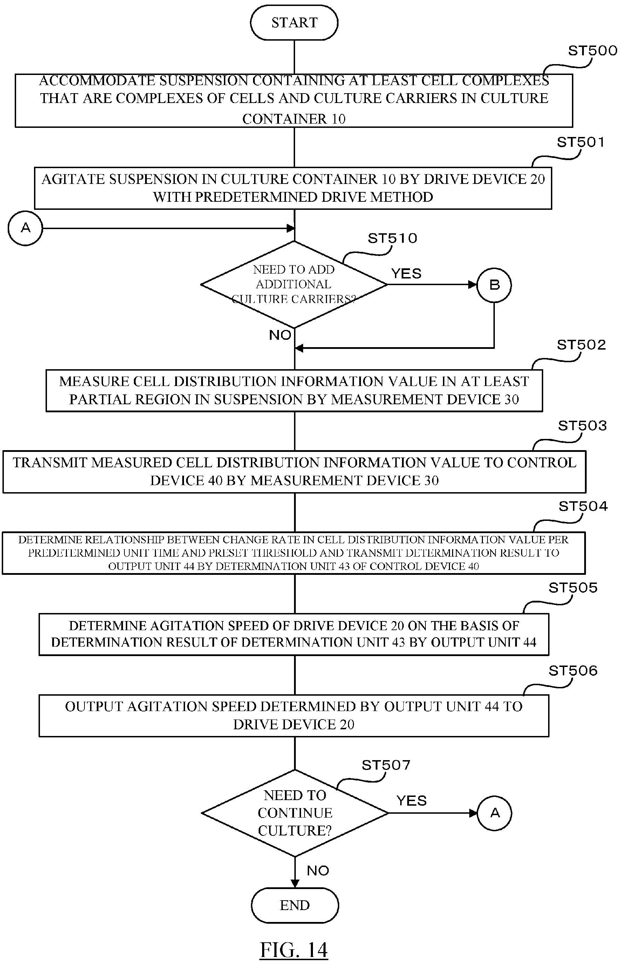

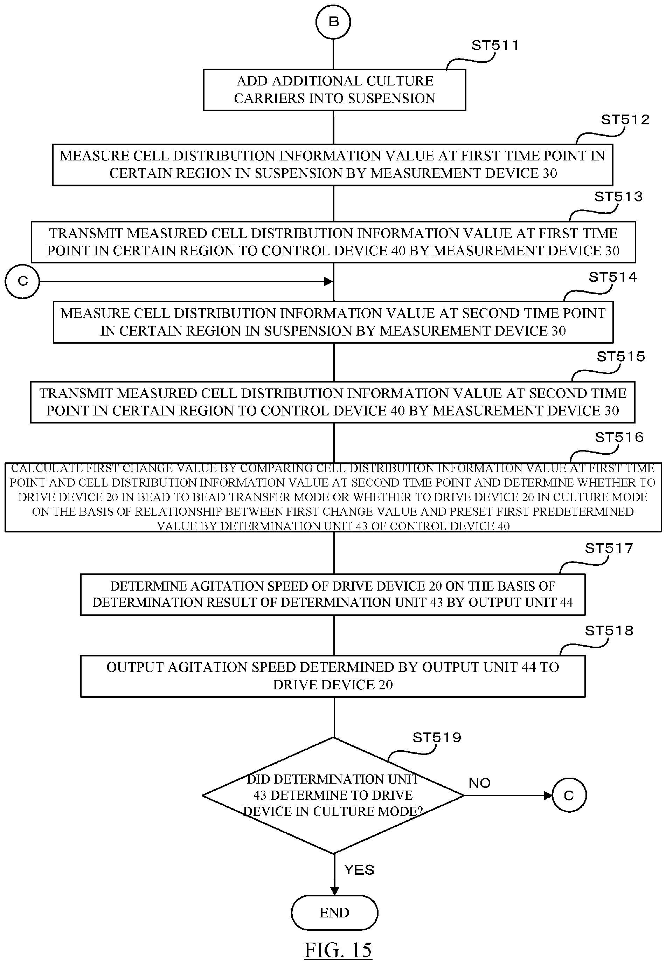

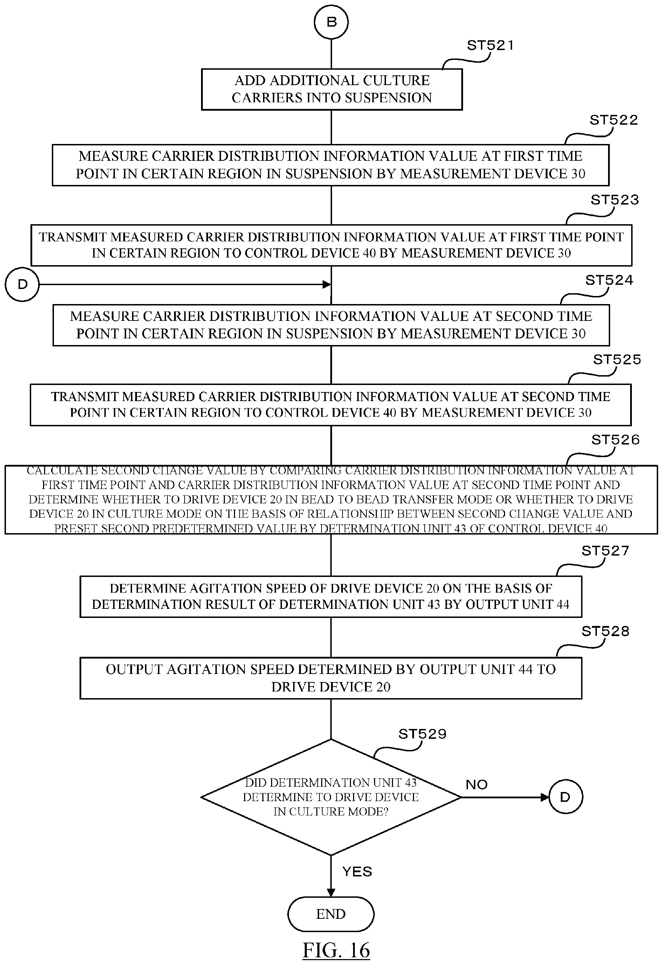

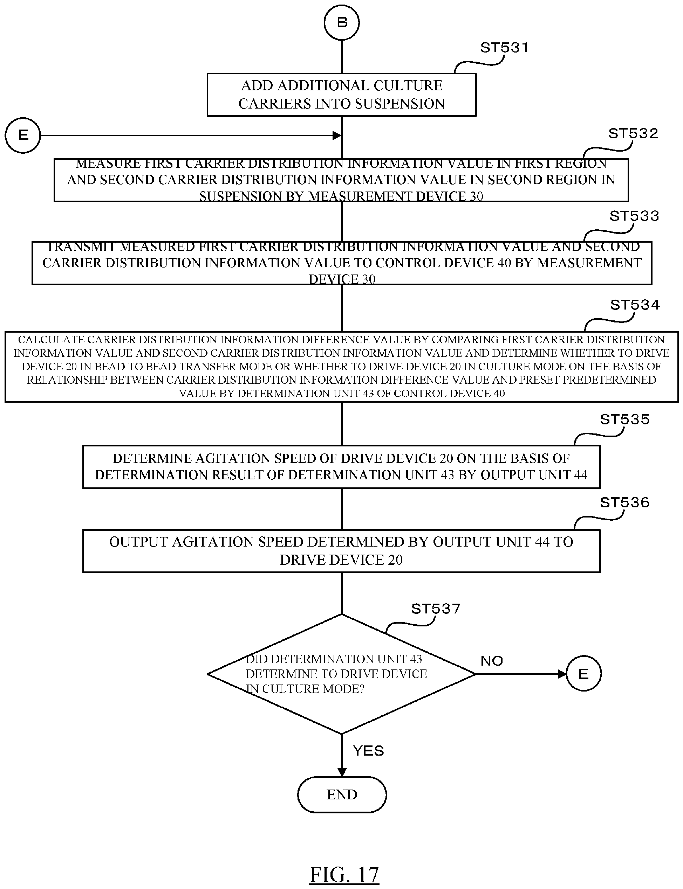

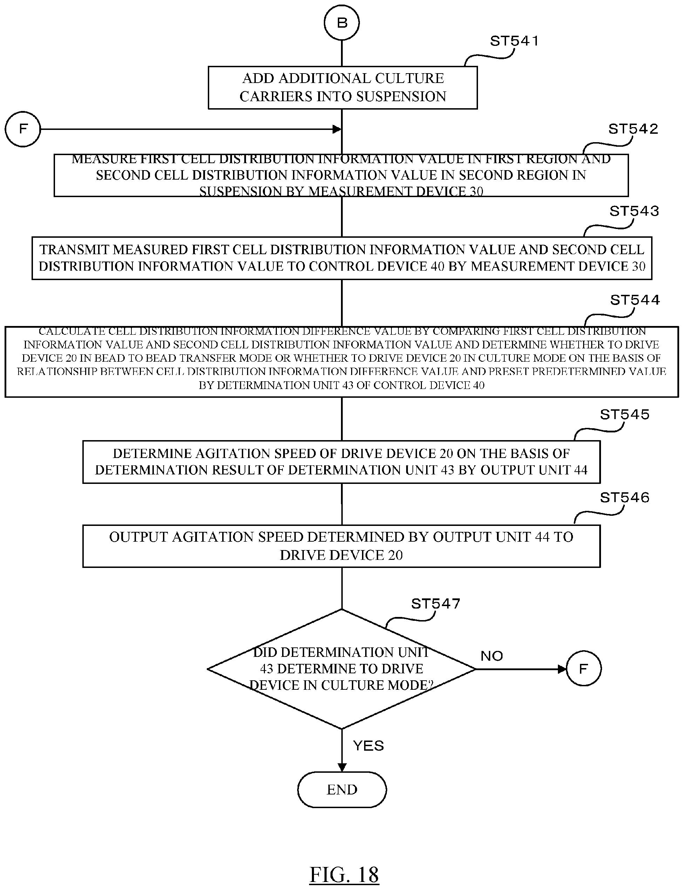

Among the series of operations in the cell culture apparatus 1 described above, the operation in the bead to bead transfer mode will be described with reference to Figs. 14 to 18. Fig. 14 is a flowchart showing an example of a series of almost all operations performed in the cell culture apparatus 1. Fig. 15 is a flowchart showing an operation related to the bead to bead transfer mode of the cell culture apparatus 1 according to the first variation. Fig. 16 is a flowchart showing an operation related to the bead to bead transfer mode of the cell culture apparatus 1 according to the second variation. Fig. 17 is a flowchart showing an operation related to the bead to bead transfer mode of the cell culture apparatus 1 according to the third variation. Fig. 18 is a flowchart showing an operation related to the bead to bead transfer mode of the cell culture apparatus 1 according to the fourth variation.

First, in the cell culture apparatus 1 according to the present disclosure, a series of operations generally illustrated in Fig. 14 is executed. The bead to bead transfer of the cells can be efficiently advanced by executing the series of operations illustrated in Fig. 14, and as a result, it is possible to realize a method for manufacturing a cell group capable of culturing cells with a high yield and time efficiency.

Among the series of operations illustrated in Fig. 14, ST500 to ST507 are as described above with reference to Fig. 8, and in particular, ST502 to ST506 (and ST507) correspond to the operations in the culture mode of the cell culture apparatus 1 according to the present disclosure.

The series of operations of the cell culture apparatus 1 according to the present disclosure further includes an operation performed in the bead to bead transfer mode in addition to ST500 to ST507. In the series of operations, as illustrated in Fig. 14, in ST510, it is determined whether or not it is required to add the additional culture carriers into the culture container 10 (in the suspension). As described above, since the control device 40 (determination unit 43) can determine a time point at which the additional culture carriers are added into the culture container 10 in the culture process of the cells, the control device 40 (determination unit 43) can execute the determination related to ST510 in association with the above determination.

As long as "NO" is determined in ST510 (as long as it is determined that it is not required to add the additional culture carriers), the control device 40 of the cell culture apparatus 1 according to the present disclosure continues to drive the drive device 20 in the culture mode.

On the other hand, as long as "YES" is determined in ST510 (when it is determined that it is required to add the additional culture carriers), the control device 40 of the cell culture apparatus 1 according to the present disclosure starts to drive the drive device 20 in the bead to bead transfer mode (see "B" of Fig. 14). The control by the control device 40 in the bead to bead transfer mode includes, as an example, the first variation to the fourth variation as described above. Therefore, the operation of each variation will be described below with reference to Figs. 15 to 18. In the control device 40, as described above, two or more of the first variation to the fourth variation may be combined. In this case, two or more operations in Figs. 15 to 18 are executed simultaneously.

First, a case where the first variation is used as the bead to bead transfer mode will be described with reference to Fig. 15.

First, the bead to bead transfer mode is started on the basis of the result of determination as "YES" in ST510 described above, and at the same time, the additional culture carriers are added into the culture container 10 (into the suspension) in ST511.

Next, in ST512, the measurement device 30 (for example, the first measurement device 300 or the second measurement device 350) measures a cell distribution information value at the first time point in a certain region in the suspension.

Next, in ST513, the measurement device 30 transmits the cell distribution information value at the first time point measured in ST512 to the control device 40, and the communication unit 41 of the control device 40 receives the cell distribution information value at the first time point.

Next, in ST514, the measurement device 30 (for example, the first measurement device 300 or the second measurement device 350) measures a cell distribution information value at the second time point in a certain region in the suspension.

Next, in ST515, the measurement device 30 transmits the cell distribution information value at the second time point measured in ST514 to the control device 40, and the communication unit 41 of the control device 40 receives the cell distribution information value at the second time point.

Next, in ST516, the determination unit 43 of the control device 40 calculates a first change value by comparing the cell distribution information value at the first time point and the cell distribution information value at the second time point, and determines whether to drive the drive device 20 in the bead to bead transfer mode (whether to continue the bead to bead transfer mode) or whether to drive the drive device 20 in the culture mode (whether to transition to the culture mode) on the basis of a relationship between the first change value and a preset first predetermined value.

Next, in ST517, on the basis of the determination result of the determination unit 43, the output unit 44 determines an agitation speed of the drive device 20. For example, in a case where the determination unit 43 determines to drive the drive device 20 in the bead to bead transfer mode in ST516, the output unit 44 determines a predetermined agitation speed as needed so as to intermittently drive the drive device 20.

Next, in ST518, the agitation speed determined by the output unit 44 is output to the drive device 20 via the communication unit 41. Accordingly, the drive device 20 is driven at the agitation speed output from the output unit 44.

Thereafter, the cell culture apparatus 1 repeats each of the operations related to ST514 to ST518 until the determination unit 43 determines to drive the drive device 20 in the culture mode (ST519). On the other hand, when the determination unit 43 determines to drive the drive device 20 in the culture mode, the drive control of the drive device 20 transits from the bead to bead transfer mode to the culture mode, and returns to the operation after ST502 of Fig. 14 (and Fig. 8).

Subsequently, a case where the second variation is used in the bead to bead transfer mode will be described with reference to Fig. 16.

The second variation is generally the same as the first variation as described above. Accordingly, ST521 to ST529 illustrated in Fig. 16 correspond to ST511 to ST519 described above, respectively. The operations in the second variation are all the same as those in the first variation except that a part described as the "cell distribution information value" in the first variation is changed to the "carrier distribution information value" (and with the change, the first change value is changed to the second change value, and the first predetermined value is changed to the second predetermined value).

Subsequently, a case where the third variation is used in the bead to bead transfer mode will be described with reference to Fig. 17.

First, the bead to bead transfer mode is started on the basis of the result of determination as "YES" in ST510 described above, and at the same time, the additional culture carriers are added into the culture container 10 (in the suspension) in ST531.

When the bead to bead transfer mode according to the third variation is started, in ST532, the measurement device 30 (for example, the second measurement device 351) measures a first carrier distribution information value in the first region and a second carrier distribution information value in the second region in the suspension.

Next, in ST533, the measurement device 30 transmits the first carrier distribution information value and the second carrier distribution information value measured in ST532 to the control device 40, and the communication unit 41 of the control device 40 receives the first carrier distribution information value and the second carrier distribution information value.

Next, in ST534, the determination unit 43 of the control device 40 calculates a carrier distribution information difference value by comparing the first carrier distribution information value and the second carrier distribution information value, and determines whether to drive the drive device 20 in the bead to bead transfer mode (whether to continue the bead to bead transfer mode) or whether to drive the drive device 20 in the culture mode (whether to transition to the culture mode) on the basis of a relationship between the carrier distribution information difference value and a preset predetermined value.

Next, in ST535, on the basis of the determination result of the determination unit 43, the output unit 44 determines an agitation speed of the drive device 20. For example, in a case where the determination unit 43 determines to drive the drive device 20 in the bead to bead transfer mode in ST533, the output unit 44 determines a predetermined agitation speed as needed so as to intermittently drive the drive device 20.

Next, in ST536, the agitation speed determined by the output unit 44 is output to the drive device 20 via the communication unit 41. Accordingly, the drive device 20 is driven at the agitation speed output from the output unit 44.

Thereafter, the cell culture apparatus 1 repeats each of the operations related to ST532 to ST536 until the determination unit 43 determines to drive the drive device 20 in the culture mode (ST537). On the other hand, when the determination unit 43 determines to drive the drive device 20 in the culture mode, the drive control of the drive device 20 transits from the bead to bead transfer mode to the culture mode, and returns to the operation after ST502 of Fig. 14 (and Fig. 8).

Subsequently, a case where the fourth variation is used in the bead to bead transfer mode will be described with reference to Fig. 18.

The fourth variation is generally the same as the third variation as described above. Accordingly, ST541 to ST547 illustrated in Fig. 18 correspond to ST531 to ST537 described above, respectively. The operations in the fourth variation are all the same as those in the third variation except that the parts described as the "first carrier distribution information value", the "second carrier distribution information value", and the "carrier distribution information difference value" in the third variation are changed to the "first cell distribution information value", the "second cell distribution information value", and the "cell distribution information difference value", respectively.

As described above, various embodiments have been exemplified, but the above embodiments are merely examples and are not intended to limit the scope of the invention. The embodiments described above can be implemented in various other forms, and various omissions, substitutions, and changes can be made without departing from the gist of the invention. Each configuration, shape, size, length, width, thickness, height, number, and the like can be appropriately changed or combined and implemented. In various embodiments described in the present disclosure, a predetermined manual process may be interposed as long as it is possible to culture cells with a high yield and time efficiency.

In the present disclosure, a range of numerical values indicated by "to" represents a range including numerical values described before and after "to" as the minimum value and the maximum value, respectively. In the range of the numerical values described in stages in the present disclosure, an upper limit value or a lower limit value of a range of numerical values of a certain stage can be arbitrarily combined with an upper limit value or a lower limit value of a range of numerical values of another stage. In the present specification, the term "step" includes not only an independent step, but also a step that cannot be clearly distinguished from other steps as long as an intended action of the step is achieved.

The present disclosure is based on the following Japanese patent application, which benefits from the priority of the Japanese patent application. The entire contents of the following Japanese patent application are incorporated into the present disclosure by reference.
(1) Japanese Patent Application No. 2020-161197, filed on September 25, 2020, titled "Cell culture apparatus and method for manufacturing cell group"

All documents, patent applications, and technical standards described in the present disclosure are incorporated into the present disclosure by reference by reference in their entirety.

## Claims

1. A cell culture apparatus, comprising:
a culture container that accommodates a suspension containing at least masses of cells and adherends to which the cells adhere;
a drive device that agitates the suspension;
a measurement device that measures a mass distribution information value related to the masses in at least one partial region in the suspension; and
a control device that controls driving of the drive device on the basis of the mass distribution information value measured by the measurement device.

2. The cell culture apparatus according to claim 1, comprising:
a culture container that accommodates a suspension containing at least cell complexes that are complexes of cells and culture carriers;
a drive device that agitates the suspension;
a measurement device that measures at least one of a cell distribution information value related to the cells and a carrier distribution information value related to the culture carriers in at least one partial region in the suspension; and
a control device that controls driving of the drive device on the basis of at least one of the cell distribution information value and the carrier distribution information value measured by the measurement device.

3. The cell culture apparatus according to claim 2, wherein
in a case where additional culture carriers as new culture carriers are added into the culture container during a culture process of the cells, the control device
controls driving of the drive device on the basis of at least one of a first change value calculated by comparing the cell distribution information value at a first time point after the addition of the additional culture carriers and the cell distribution information value at a second time point after the first time point, and a second change value calculated by comparing the carrier distribution information value at the first time point and the carrier distribution information value at the second time point.

4. The cell culture apparatus according to claim 2 or 3, wherein
in a case where additional culture carriers as new culture carriers are added into the culture container during a culture process of the cells, the control device
controls driving of the drive device on the basis of a cell distribution information difference value calculated by a comparison operation between a first cell distribution information value in a first region in the at least one partial region after the addition of the additional culture carriers and a second cell distribution information value in a second region in the at least one partial region.

5. The cell culture apparatus according to any one of claims 2 to 4, wherein
in a case where additional culture carriers as new culture carriers are added into the culture container during a culture process of the cells, the control device
controls driving of the drive device on the basis of a carrier distribution information difference value calculated by a comparison operation between a first carrier distribution information value in a first region in the at least one partial region after the addition of the additional culture carriers and a second carrier distribution information value in a second region in the at least one partial region.

6. The cell culture apparatus according to any one of claims 2 to 5, wherein
the measurement device includes
a first measurement device including an electrode that forms an electric field in the at least one partial region and a capacitance measurement unit that measures a capacitance in the electric field, and
the first measurement device measures the cell distribution information value in the at least one partial region on the basis of the capacitance.

7. The cell culture apparatus according to any one of claims 2 to 6, wherein
the measurement device includes
a second measurement device including an imaging unit that images the at least one partial region and acquires one or more images, and
the second measurement device measures at least one of the cell distribution information value and the carrier distribution information value in the at least one partial region on the basis of the images.

8. The cell culture apparatus according to claim 7, wherein the second measurement device measures the first cell distribution information value on the basis of the images in the first region, and measures the second cell distribution information value on the basis of the images in the second region.

9. The cell culture apparatus according to claim 7, wherein the second measurement device measures the first carrier distribution information value on the basis of the images in the first region, and measures the second carrier distribution information value on the basis of the images in the second region.

10. The cell culture apparatus according to any one of claims 2 to 9, wherein the cell distribution information value is the number of living cells or a concentration of living cells among the cells in the suspension in the at least one partial region.

11. The cell culture apparatus according to any one of claims 2 to 10, wherein the carrier distribution information value is the number of culture carriers or a concentration of the culture carriers in the suspension in the at least one partial region.

12. The cell culture apparatus according to claim 3, wherein
the control device
drives the drive device in a first mode until the first change value reaches a preset first predetermined value, and
drives the drive device in a second mode different from the first mode after the first change value reaches the first predetermined value.

13. The cell culture apparatus according to claim 3, wherein
the control device
drives the drive device in a first mode until the second change value reaches a preset second predetermined value, and
drives the drive device in a second mode different from the first mode after the second change value reaches the second predetermined value.

14. The cell culture apparatus according to claim 4, wherein
the control device
drives the drive device at an agitation speed in a first mode until the cell distribution information difference value reaches a preset predetermined value, and
drives the drive device at an agitation speed in a second mode different from the first mode after the cell distribution information difference value reaches the predetermined value.

15. The cell culture apparatus according to claim 5, wherein
the control device
drives the drive device at an agitation speed in a first mode until the carrier distribution information difference value reaches a preset predetermined value, and
drives the drive device at an agitation speed in a second mode different from the first mode after the carrier distribution information difference value reaches the predetermined value.

16. The cell culture apparatus according to any one of claims 12 to 15, wherein the control device intermittently drives the drive device in the first mode.

17. The cell culture apparatus according to any one of claims 2 to 16, wherein the drive device is a stirrer or a shaker that stirs the suspension.

18. The cell culture apparatus according to any one of claims 2 to 17, wherein a volume of the culture container is 2.0 L or more.

19. The cell culture apparatus according to any one of claims 2 to 18, wherein the at least one partial region includes at least one of a central portion of the culture container in a depth direction in the suspension and a bottom surface portion of the culture container in the suspension.

20. The cell culture apparatus according to claim 4 or 5, wherein
the first region is a central portion of the culture container in a depth direction in the suspension, and
the second region is a bottom surface portion of the culture container in the suspension.

21. The cell culture apparatus according to any one of claims 2 to 20, wherein
the control device
determines a time point at which the cell distribution information value reaches a predetermined set value after a start of culture of the cells as a time point at which additional culture carriers as new culture carriers are added into the culture container.

22. A method for manufacturing a cell group by a culture method, the culture method comprising:
accommodating a suspension containing at least masses of cells and adherends to which the cells adhere in a culture container;
agitating the suspension with a drive device;
measuring a mass distribution information value related to the masses in at least one partial region in the suspension;
determining an agitation speed of the drive device on the basis of the measured mass distribution information value; and
outputting the determined agitation speed to the drive device.

23. The method for manufacturing the cell group according to claim 22, the method comprising:
accommodating a suspension containing at least cell complexes that are complexes of cells and culture carriers in a culture container;
agitating the suspension with a drive device;
measuring at least one of a cell distribution information value related to the cells and a carrier distribution information value related to the culture carriers in at least one partial region in the suspension;
determining an agitation speed of the drive device on the basis of at least one of the measured cell distribution information value and carrier distribution information value; and
outputting the determined agitation speed to the drive device.

24. The method for manufacturing the cell group according to claim 23, wherein
in a case where additional culture carriers as new culture carriers are added into the culture container during a culture process of the cells, the agitation speed is
determined on the basis of at least one of a first change value calculated by comparing the cell distribution information value at a first time point after the addition of the additional culture carriers and the cell distribution information value at a second time point after the first time point, and a second change value calculated by comparing the carrier distribution information value at the first time point and the carrier distribution information value at the second time point.

25. The method for manufacturing the cell group according to claim 23 or 24, wherein
in a case where additional culture carriers as new culture carriers are added into the culture container during a culture process of the cells, the agitation speed is
determined on the basis of a cell distribution information difference value calculated by a comparison operation between a first cell distribution information value in a first region among a plurality of regions after the addition of the additional culture carriers and a second cell distribution information value in a second region among the plurality of regions.

26. The method for manufacturing the cell group according to any one of claims 23 to 25, wherein
in a case where additional culture carriers as new culture carriers are added into the culture container during a culture process of the cells, the agitation speed is
determined on the basis of a carrier distribution information difference value calculated by a comparison operation between a first carrier distribution information value in a first region among a plurality of regions after the addition of the additional culture carriers and a second carrier distribution information value in a second region among the plurality of regions.
